# EUROPEAN PATENT APPLICATION

(11) **EP 4 159 736 A1**
(43) Date of publication of application: **05.04.2023**
(21) Application number: 21809085.0
(22) Date of filing: 24.05.2021
(51) Int. Cl.: C07D 487/10, A61K 31/4439, A61P 35/00

(54) **NOVEL TRICYCLIC AROMATIC HETEROCYCLIC COMPOUND AND PREPARATION METHOD THEREFOR, PHARMACEUTICAL COMPOSITION AND USE THEREOF**

(30) Priority: 22.05.2020 CN 202010443498
(71) Applicant: Shanghai Longwood Biopharmaceuticals Co., Ltd., Shanghai 201108 (CN)
(72) Inventor: WANG, Zhe, Shanghai 201108 (CN); BAI, Haiyun, Shanghai 201108 (CN); QIAN, Anran, Shanghai 201108 (CN); LI, Deliang, Shanghai 201108 (CN)
(74) Representative: Lavoix
(86) International application number: PCT/CN2021/095604
(87) International publication number: WO 2021/233454

(57) **Abstract**

Provided in the present invention are a novel tricyclic aromatic heterocyclic compound and a preparation method therefor, a pharmaceutical composition and a use thereof. Specifically, provided in the present invention is a compound as shown in formula I below, or an optical isomer, a hydrate, a solvate thereof, or a pharmaceutically acceptable salt thereof. The definitions of each group are as specified in the description. The compound of formula I can be used for treating diseases related to a PD-1/PD-L1 signaling pathway.

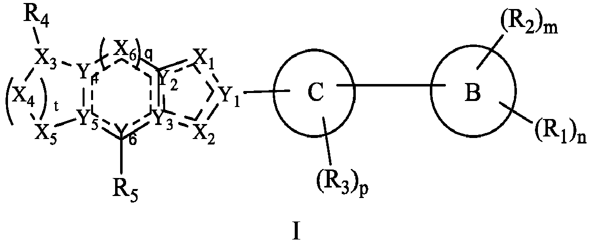

## Description

### TECHNICAL FIELD

The present invention relates to the field of small molecule drugs, and specifically, the present invention provides a small molecule compound that can be used to treat diseases related to PD-1/PD-L1 signaling pathway.

### BACKGROUND OF THE INVENTION

The immune system plays a vital role in controlling and curing many diseases, such as various cancers, diseases caused by viruses, etc. But the cancer cells can evade or inhibit the immune system in some way to proliferate rapidly. One way is to alter the activator molecules and inhibitory molecules expressed on immune cells. Blocking inhibitory immune checkpoints, such as PD-1, has been proven to be a very effective approach to suppressing cancer cells.

PD-1 is programmed cell death protein-1, also known as CD279. It is mainly expressed in activated T cells and B cells, and its function is to inhibit the activation of cells, which is a normal homeostatic mechanism of the immune system, because excessive T/B cell activation can cause autoimmune diseases, PD-1 is a protective wall of our body. PD-1 is a type I transmembrane glycoprotein composed of 268 amino acids, and its structure mainly includes the outer immunoglobulin variable domain, the hydrophobic transmembrane domain and the intracellular domain. The intracellular domain contains two phosphorylation sites, and they are located in the immunoreceptor tyrosine inhibitory motif and the immunoreceptor tyrosine switching motif, respectively, which also proves that PD-1 can negatively regulate T cell receptor-mediated signal. PD-1 has two ligands, PD-L1 and PD-L2, which differ in expression way. PD-L1 is up-regulated in a variety of tumor cells, and it binds to PD-1 on T cells, inhibits T cell proliferation and activation, makes T cells in a state of inactivation, and ultimately induces immune escape.

PD-1/PD-L1 plays an inverse immunomodulatory role. When PD-1 binds to PD-L1, it can cause tyrosine polyphosphorylation in the immunoreceptor tyrosine switch motif domain of T cells, and the phosphorylated tyrosine can bind to the phosphatase protein tyrosinase 2 and protein tyrosinase 1, which can impede the activation of extracellular signal-regulated kinase and also block the activation of phosphatidylinositol 3-kinase (PI3K) and serine-threonine protein kinase (Akt), thereby inhibiting T lymphocyte proliferation and the secretion of related cytokines. The PD-1/PD-L1 signal inhibits the activation and proliferation of T cells, and at the same time, it can also induce the secretion of cytokines interleukin 2, interferon γ and IL-10. In addition, PD-1/PD-L1 signaling also has a similar immune function on B cells. After PD-1 binds to B cell antigen receptors, the PD-1 cytoplasmic domain interacts with tyrosinase containing a protein tyrosinase 2 binding site, thereby hindering B cell activation.

The immunotherapy based on PD-1/PD-L1 is a new generation of immunotherapy that has attracted much attention. In recent years, a series of surprising findings have confirmed that PD-1/PD-L1 inhibitors have strong antitumor activity against a variety of tumors. Currently available PD-1/PD-L1 antibody regulators include BMS's Ninolumab, Merck's Lambrolizumab and Roche's Atezolizumab. In addition, there are many PD-1/PD-L1 antibody regulators in development, including CureTech's Pidilizumab, GSK's AMP-224 and AstraZeneca's MEDI-4736.

Although tumor immunotherapy is considered to be a new generation of revolution in cancer treatment after targeted therapy, the PD-1 monoclonal antibodies currently on the market and under development have their own shortcomings. They can only be administered by injection and cannot be taken orally. They are unstable in the body, are easily decomposed by proteases, and are prone to immune cross-reaction. Moreover, it is difficult to purify and the production cost is high. Therefore, small molecule regulator of PD-1/PD-L1 interaction is a better option for tumor immunotherapy.

In summary, there is an urgent need in the art to develop novel small-molecule regulator of PD-1/PD-L1 interaction.

### SUMMARY OF THE INVENTION

The object of the present invention is to provide a novel small-molecule regulator of PD-1/PD-L1 interaction.

In the first aspect of the invention, a compound shown in Formula I, or optical isomers, hydrates, solvates thereof, or pharmaceutically acceptable salts thereof is provided: 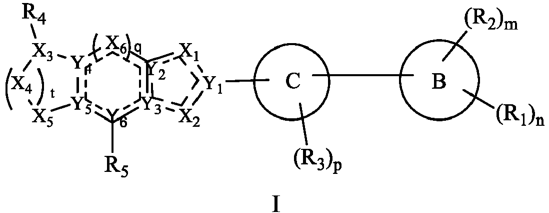
wherein, n is 0, 1, 2, or 3;
m, p, q, and t are each independently selected from 0, 1, 2, 3, or 4;
X₁, X₂, X₃, X₄, X₅ and X₆ are each independently selected from the group consisting of N, O, S, SO, SO₂, C(R)₂, or NR;
Y₁, Y₂, Y₃, Y₄, Y₅ and Y₆ are each independently selected from the group consisting of N, CH, or C; 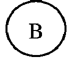
is selected from the group consisting of substituted or unsubstituted C1-C15 aryl, or substituted or unsubstituted 4-12 membered (preferably 5-7 membered) heteroaryl with 1-4 heteroatoms, substituted or unsubstituted 4-12 membered heterocyclyl, and substituted or unsubstituted 4-12 membered C5-C12 cycloalkyl; 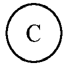
is selected from the group consisting of substituted or unsubstituted C1-C15 arylene, or substituted or unsubstituted 4-12 membered (preferably 5-7 membered) heteroarylene with 1-4 heteroatoms, substituted or unsubstituted 4-12 membered heterocyclylene, and substituted or unsubstituted 4-12 membered C5-C12 cycloalkylene;
R, R₁, R₂, R₃, R₄ and R₅ are each independently selected from the group consisting of H, -CN, trifluoromethyl, sulfonamido, nitro, hydroxyl, halogen, -S-R₈, -S(O)-R₈, -S(O)₂-R₈, substituted or unsubstituted C1-C10 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl, oxo (i.e.=O), =NRε, -CN, hydroxyl, NR_{d}Rₑ (e.g. amino), substituted or unsubstituted C1-C6 amino, substituted or unsubstituted -(C1-C6 alkylene)-NH-(C1-C6 alkylene), carboxyl, substituted or unsubstituted C6-C10 aryl, substituted or unsubstituted 5-12 membered heteroaryl with 1-3 heteroatoms, substituted or unsubstituted 3-12 membered heterocyclyl with 1-4 heteroatoms, substituted or unsubstituted 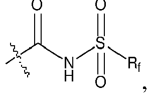 substituted or unsubstituted 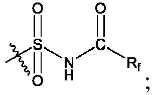 or -(L₁ₐ)ᵣ-(L₂ₐ)ₛ-(L₃ₐ)ₛ-; -C₀₋₈-O-R₈, -C₀₋₈-C(O)OR₈, -C₀₋₈-OC(O)OR₈, -C₀₋₈-NR₈R₉, -C₀₋₈-N(R₈)C(O)R₉, -C₀₋₈-C(O)NR₈R₉;
or R₁ and R₂ together with the adjacent ring atom form a 5, 6 or 7 membered carbon ring or a 5, 6 or 7 membered heterocycle, wherein the heterocycle contains 1, 2 or 3 heteroatoms selected from N, S or O; the carbon ring or heterocycle can be an aromatic ring conjugated with the Bring, or a non-aromatic ring containing unsaturated bond, or a saturated ring;
R₈ and R₉ are each independently selected from the group consisting of H, hydroxyl, substituted or unsubstituted C1-C10 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl, oxo (i.e.=O), =NR_{f}, -CN, hydroxyl, NR_{d}Rₑ (e.g. amino), substituted or unsubstituted C1-C6 amino, substituted or unsubstituted -(C1-C6 alkylene)-NH-(C1-C6 alkylene), carboxyl, substituted or unsubstituted C6-C10 aryl, substituted or unsubstituted 5-12 membered heteroaryl with 1-3 heteroatoms, substituted or unsubstituted 3-12 membered heterocyclyl with 1-4 heteroatoms, substituted or unsubstituted 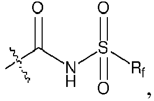 substituted or unsubstituted 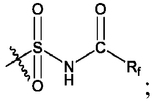 or -(L₁ₐ)ᵣ-(L₂ₐ)ₛ-(L₃ₐ)ₛ-;
each L₁ₐ is independently selected from the group consisting of chemical bond, substituted or unsubstituted C₁-C₇ alkylene, substituted or unsubstituted C2-C4 alkenylene, substituted or unsubstituted C2-C4 alkynylene, -S-, -O-, substituted or unsubstituted -NH-, -S(O)-, -S(O)₂-;
L₂ₐ is selected from the group consisting of substituted or unsubstituted C6-C12 arylene, substituted or unsubstituted 5-12 membered heteroarylene with 1-3 heteroatoms, substituted or unsubstituted C3-C8 cycloalkylene, substituted or unsubstituted 5-10 membered heterocyclylene with 1-3 heteroatoms;
L₃ₐ is selected from the group consisting of substituted or unsubstituted C1-C10 alkyl, C1-C10 aryl, -CN, hydroxyl, amino, carboxyl, -CO-NH-SO₂-R_{g}, -NH-SO₂-R_{g}, -SO₂-NH-CO-R_{g};
r is 1, 2, 3, 4, 5, or 6;
s is 0, 1, or 2;
R_{d}, Rₑ and R_{g} are each independently selected from the group consisting of H, substituted or unsubstituted C₁-C₆ alkyl, substituted or unsubstituted C₃-C₁₀ cycloalkyl, substituted or unsubstituted C₆-C₁₀ aryl; or R_{d} and Rₑ together form a substituted or unsubstituted 5-10 membered heterocyclyl with 1-3 heteroatoms selected from N, S and O;
R_{f} is selected from the group consisting of H, substituted or unsubstituted C₁-C₆ alkyl, substituted or unsubstituted C₆-C₁₀ aryl, substituted or unsubstituted C₆-C₁₀ heteroaryl, cyano, - C(=O)-NR_{d}Rₑ, -C(=O)-substituted or unsubstituted C₁-C₆ alkoxy, -C(=O)-substituted or unsubstituted C₁-C₆ alkyl, -C(=O)-substituted or unsubstituted C₃-C₁₀ cycloalkyl, -C(=O)-substituted or unsubstituted C₂-C₆ alkenyl, -C(=O)-substituted or unsubstituted C₂-C₆ alkynyl;
unless otherwise specified, the "substituted" means being substituted by one or more (such as 2, 3, 4, etc.) substituents selected from the group consisting of halogen, including but not limited to F, Cl, Br, -CH₂Cl, -CHCl₂, -CCl₃, -CH₂F, -CHF₂, -CF₃, oxo, -CN, hydroxyl, amino, C1-C6 alkylamino, carboxyl, -NHAc, or substituted or unsubstituted groups which are selected from C₁-C₆ alkyl, C₁-C₆ alkoxy, C₆-C₁₀ aryl, C₃-C₈ cycloalkyl, halogenated C₆-C₁₀ aryl, 5-10 membered heteroaryl with 1-3 heteroatoms selected from N, S and O, and 5-10 membered heterocyclyl with 1-3 heteroatoms selected from N, S and O, and the substituent of which is selected from halogen, hydroxyl, carboxyl, cyano, C₁-C₆ alkoxy, and C₁-C₆ alkylamino;
among the above formula, any of the heteroatoms is selected from the group consisting of B, P, N, S and O.

In another preferred embodiment, R₄ is 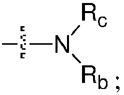 wherein R_{b} and R_{c} R_{d} are each independently selected from the group consisting of H, substituted or unsubstituted C₁-C₈ alkyl; or R_{b} and R_{c} together with adjacent N atom form a substituted or unsubstituted 4-10 membered heterocyclyl with 1-3 heteroatoms selected from N, S and O.

In another preferred embodiment, 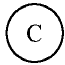 is a divalent group formed by a ring selected from the group consisting of 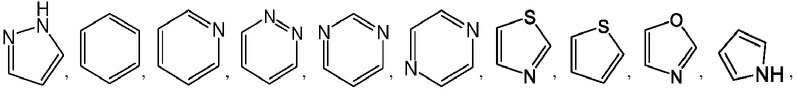 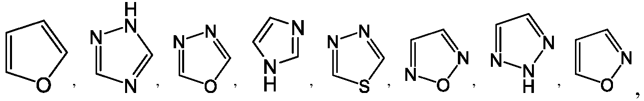 wherein bonding position of the ring can be N or C.

In another preferred embodiment, R₃ is selected from the group consisting of methyl, -F, Cl, Br, -CH₂Cl, -CHCl₂, -CCl₃, -CH₂F, -CHF₂, -CF₃, -CN, hydroxyl, amino.

In another preferred embodiment, 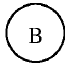 is selected from the group consisting of 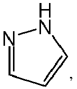 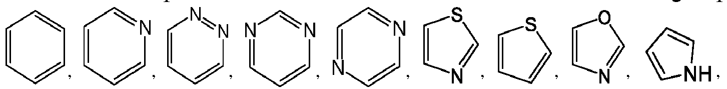 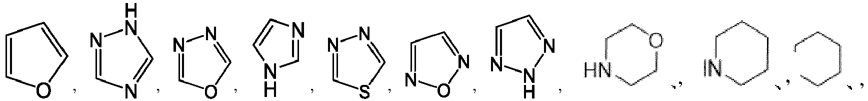 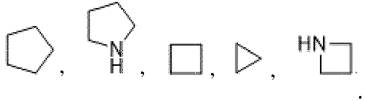 and R₁ and R₂ are each independently selected from the group consisting of H, halogen, substituted or unsubstituted C1-C10 alkyl.

In another preferred embodiment, 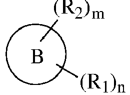 is selected from the group consisting of:

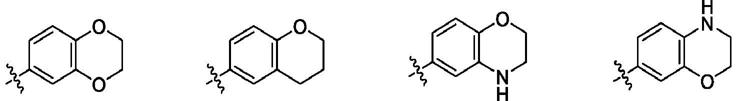

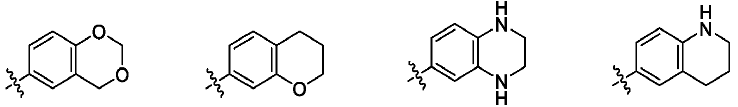

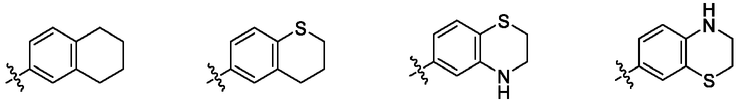

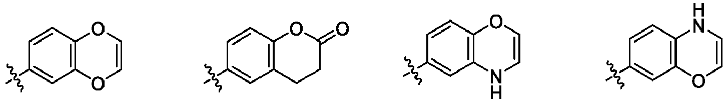

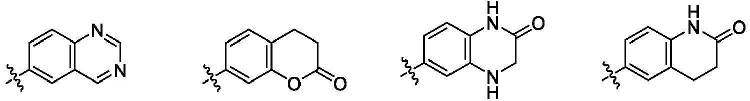

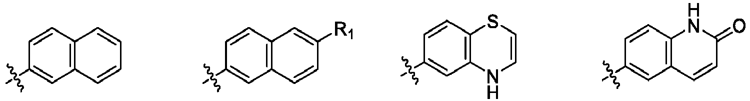

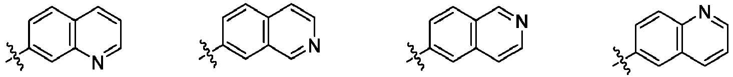

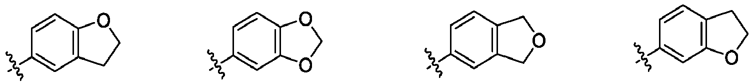

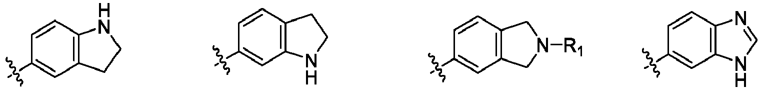

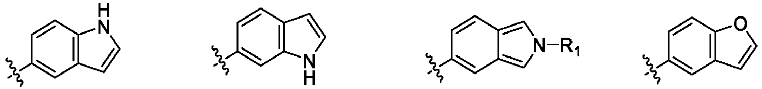

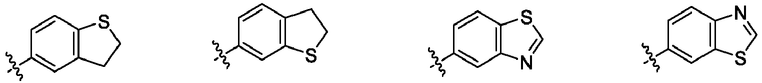

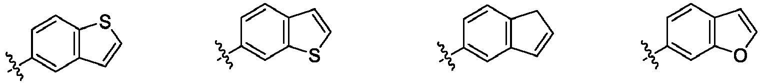

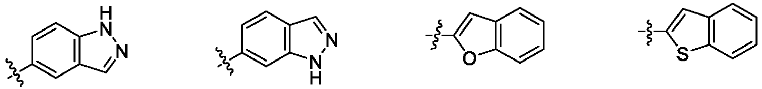

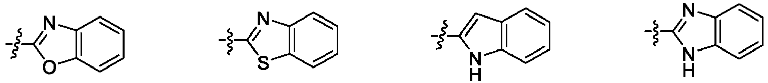

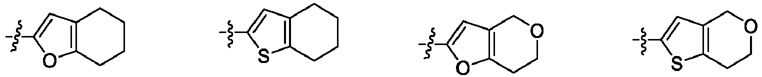

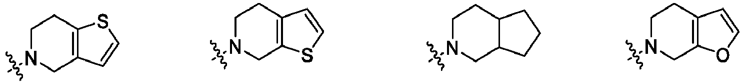

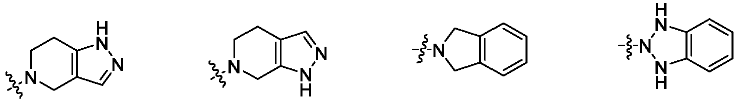

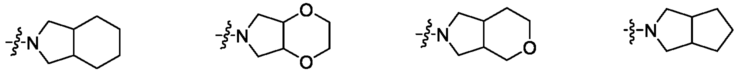

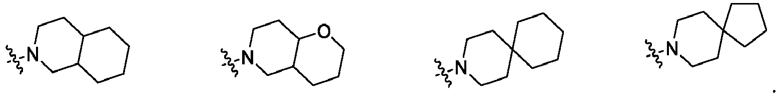

In another preferred embodiment, 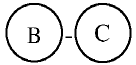 is the structure shown below:

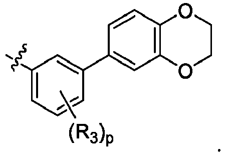

In another preferred embodiment, 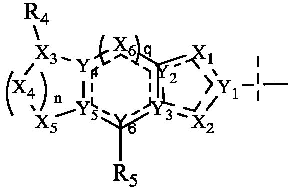 is the structure shown below:

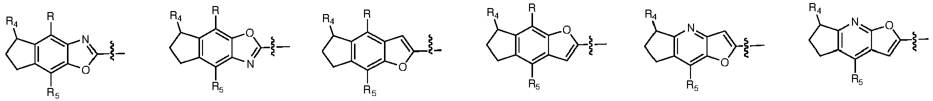

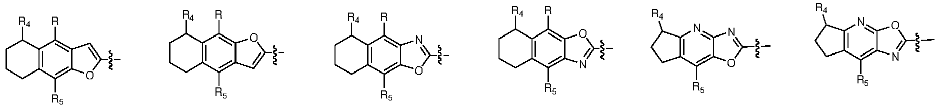

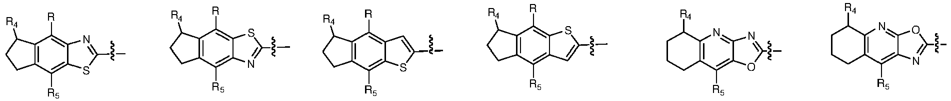

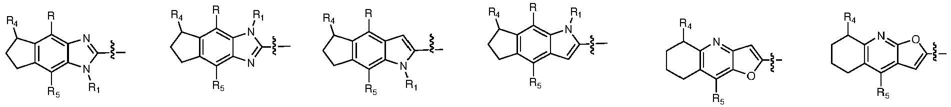

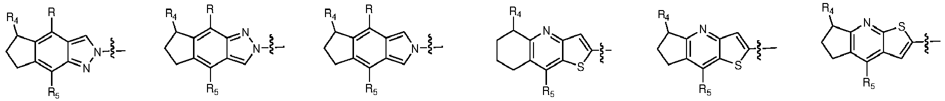

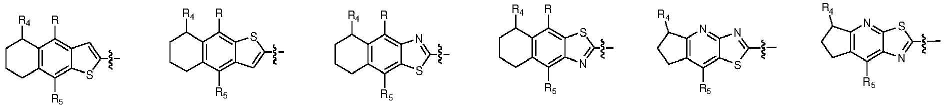

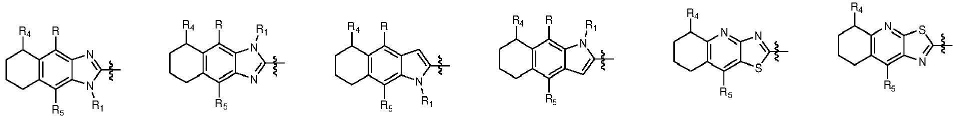

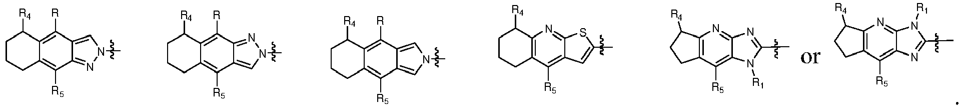

In another preferred embodiment, the compound is selected from the group consisting of:

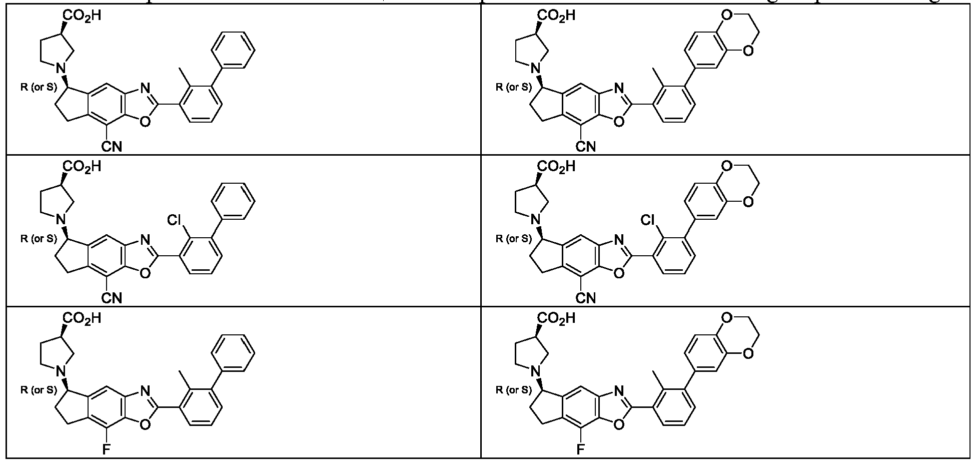

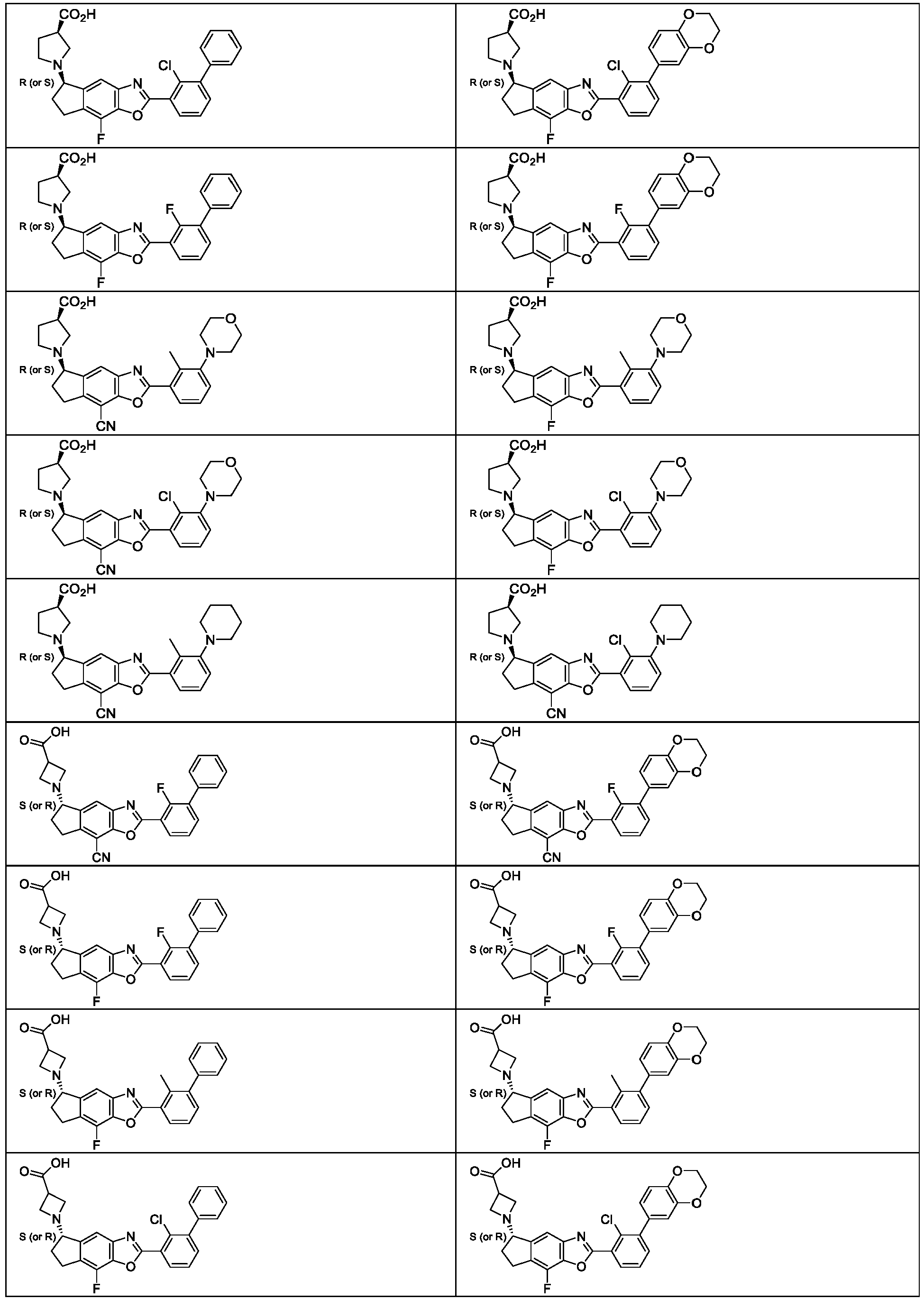

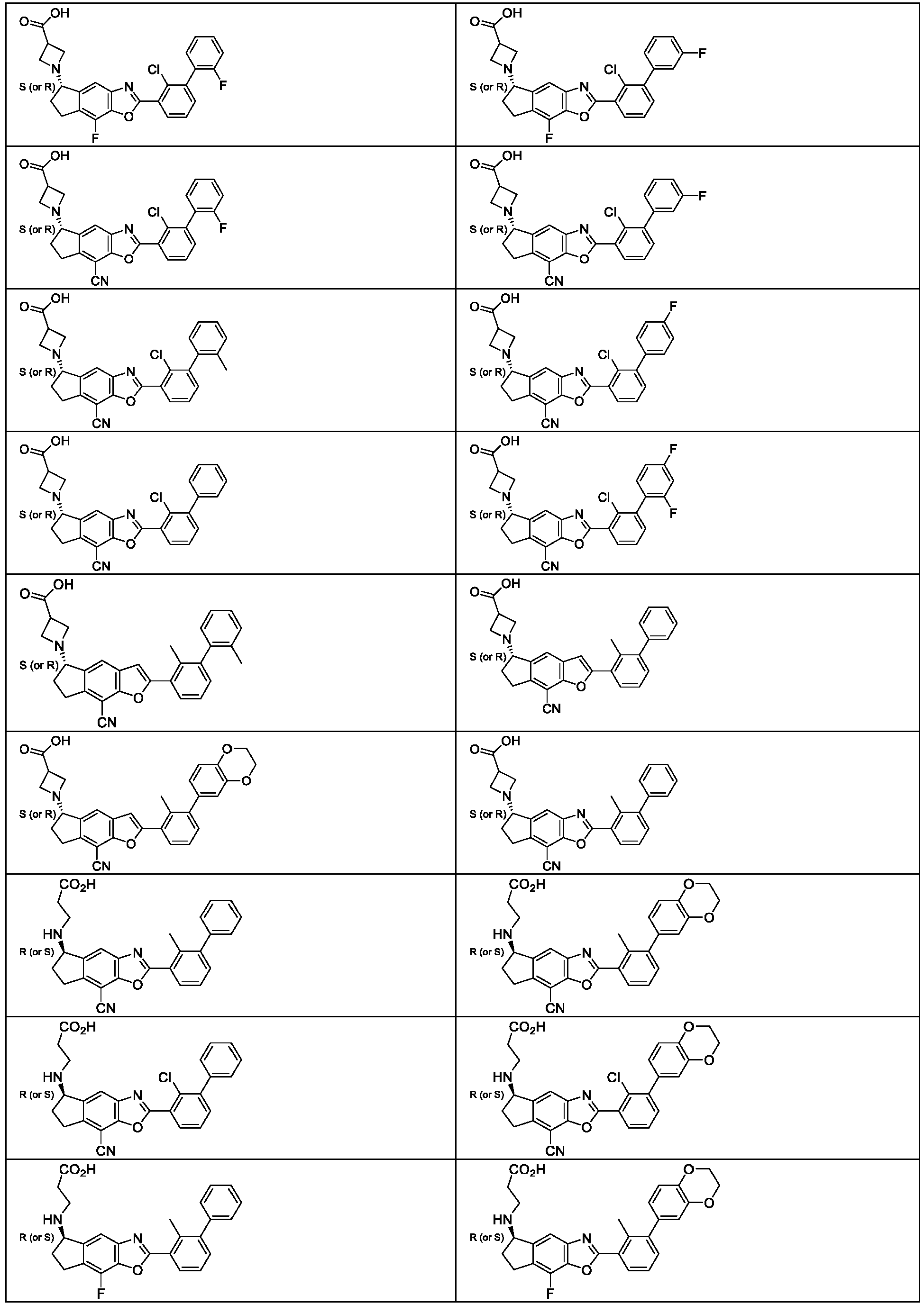

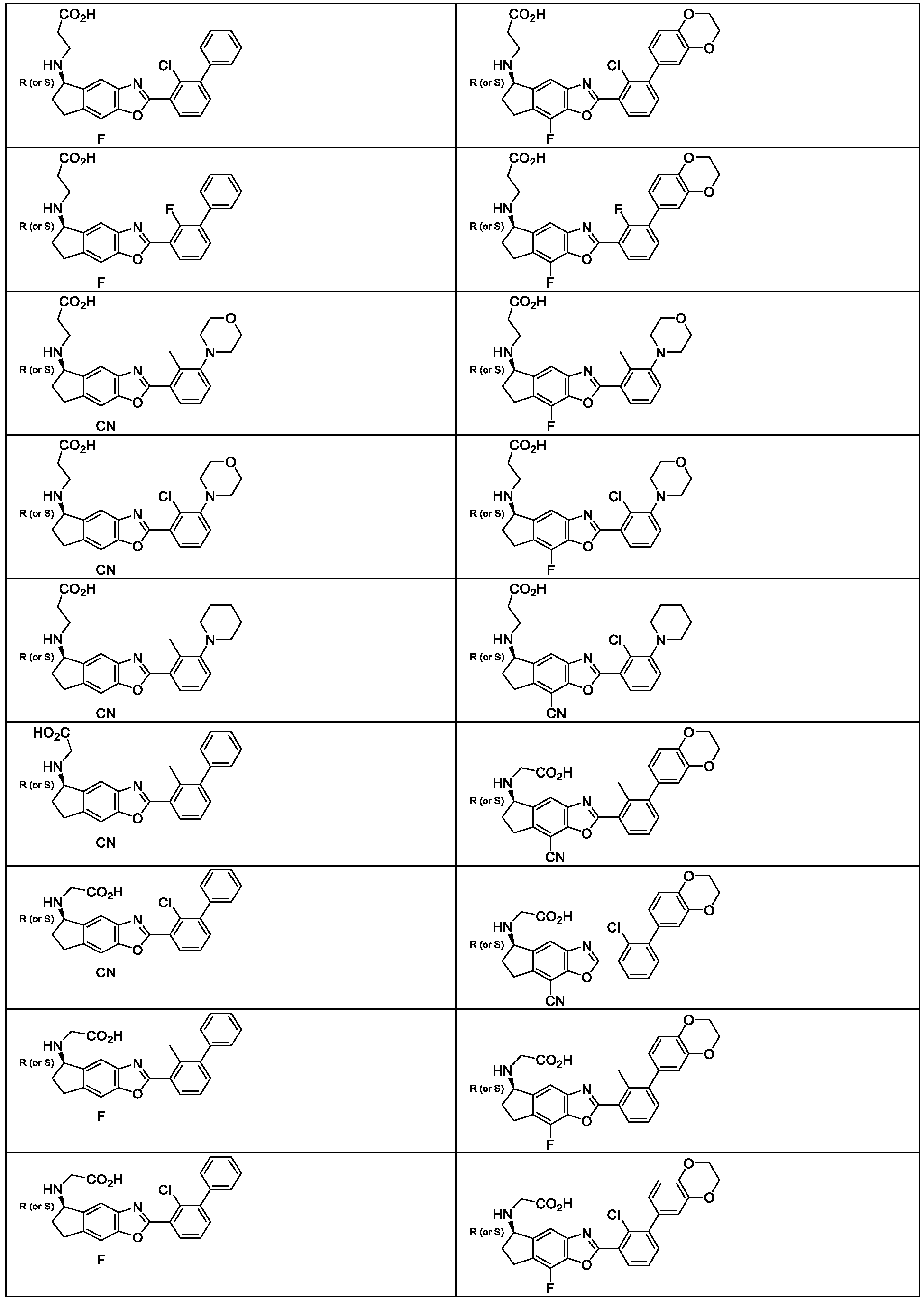

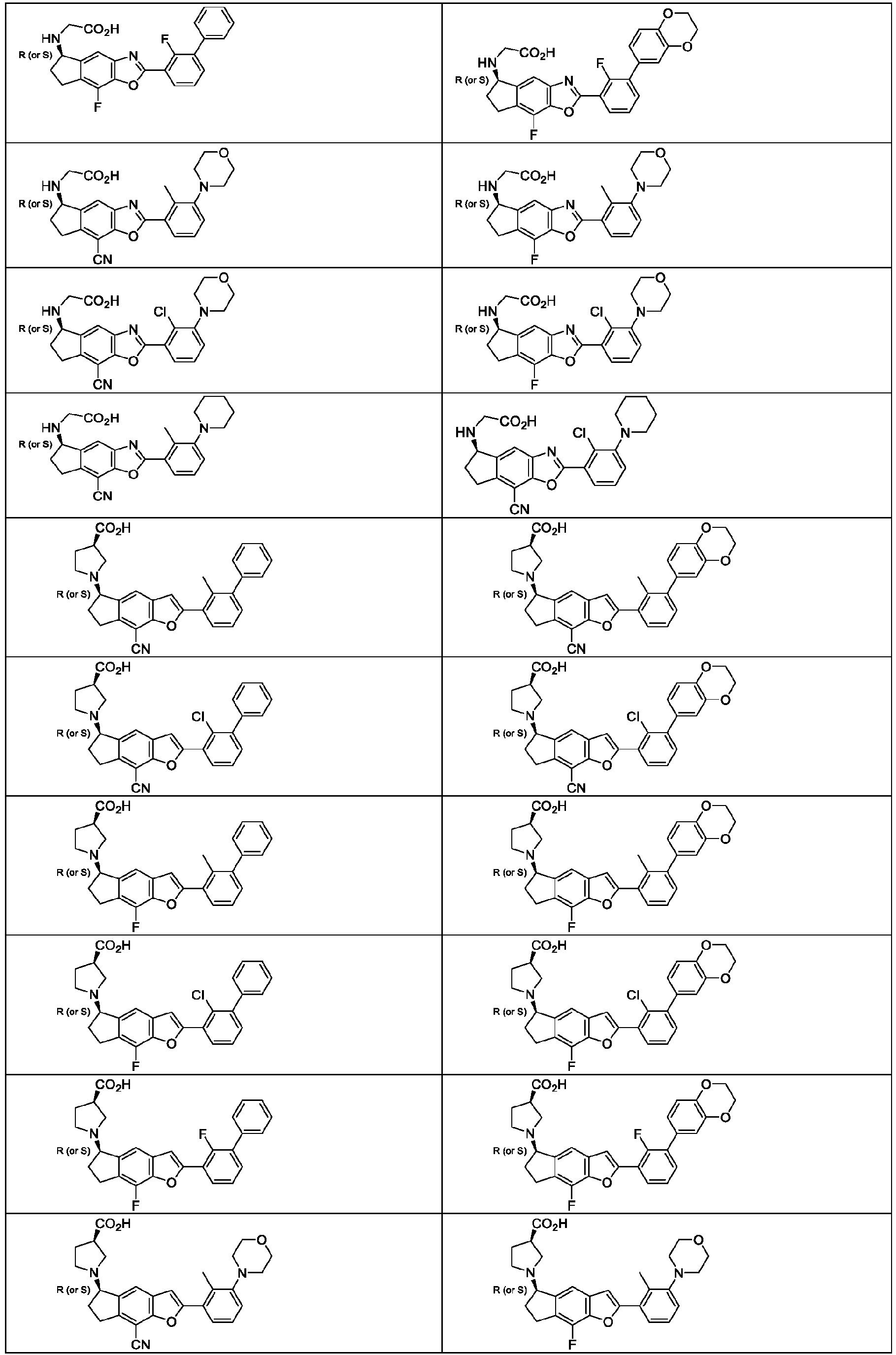

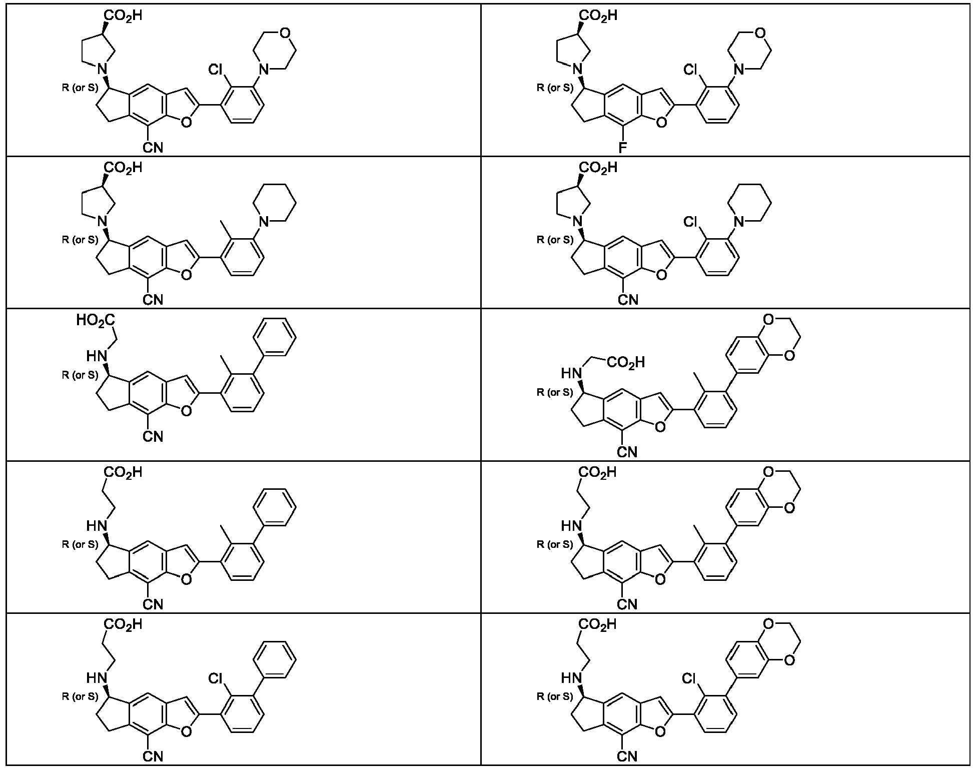

The second aspect of the invention provides a method for preparing compounds of formula I as described in the first aspect of the invention, and the method comprises the steps selected from the synthesis scheme 1 or 2:

### Synthesis scheme 1:

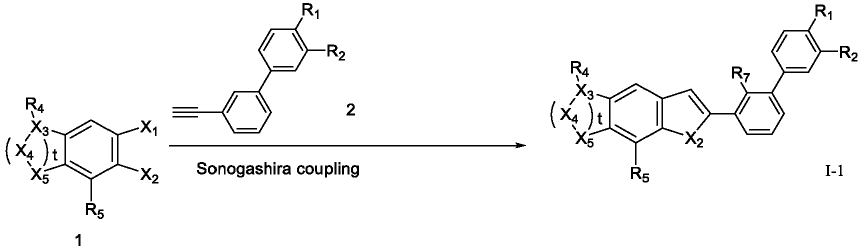

(a) providing the target product **1-1** by Sonogashira coupling reaction catalyzed by palladium catalyst by using intermediate **1** and **2** as raw materials;

Preferably, the preparation method of intermediate **1** is as follows:

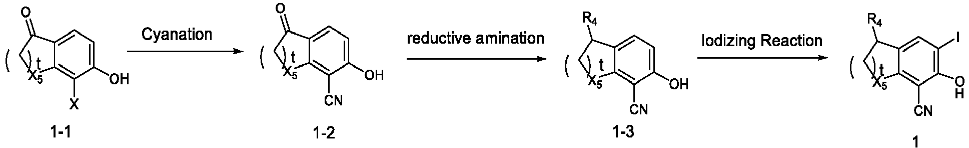

Providing intermediate **1-2** by coupling reaction with palladium catalyst and ligand using compound **1-1** as raw material;
(b) Providing intermediate **1-3** by first condensation reaction with primary and secondary amines under the catalysis of Lewis acid using **1-2** as the raw material, then conducting reduction reaction by adding appropriate reducing agent; or obtaining the above by directly reductive amination reaction in the presence of reducing agent;
(c) Providing intermediate **1** by halogenation reaction catalyzed by Lewis acid using **1-3** as raw material;

### Synthetic scheme 2:

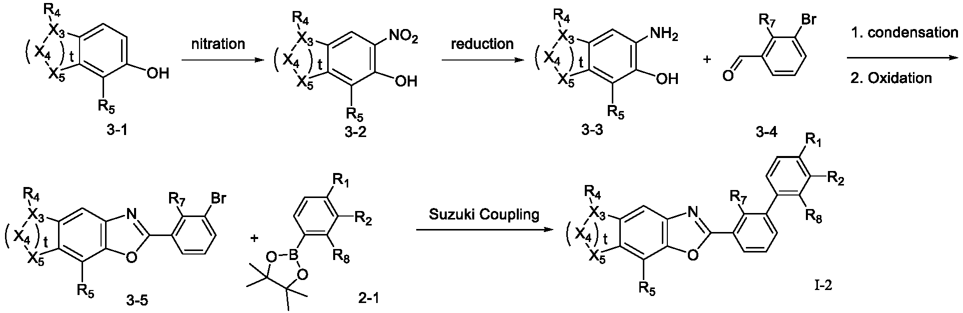

(a) Providing intermediate **3-2** by using nitrating agent (such as concentrated sulfuric acid/NaNO₃, concentrated sulfuric acid/fuming nitric acid, etc.) to react with compound **3-1;**
(b) Providing intermediate **3-3** by reducing **3-2** under reductive conditions (Pd-C/H₂; zinc powder/ammonium chloride; iron powder/acetic acid, etc.);
(c) Providing intermediate **3-5** by condensing **3-3** and **3-4** with condensation agent, then oxidating with appropriate oxidant (such as DDQ);
(d) Providing intermediate **1-2** by coupling **3-5** and **3-6** with catalyst and ligand.

The third aspect of the present invention provides a pharmaceutical composition, which comprises (1) the compound according to the first aspect of the present invention or stereoisomers or tautomers thereof, or pharmaceutically acceptable salts, hydrates or solvates thereof; (2) pharmaceutically acceptable carriers.

The fourth aspect of the present invention provides a use of the compound according to the first aspect of the present invention or stereoisomers or tautomers thereof, or pharmaceutically acceptable salts, hydrates or solvates thereof, or the pharmaceutical composition according to the third aspect of the present invention, for the preparation of a pharmaceutical composition for preventing and/or treating diseases related to the activity or expression of PD-1/PD-L1.

The fifth aspect of the present invention provides a PD-1/PD-L1 regulator, the regulator comprises the compound according to the first aspect of the present invention, or stereoisomers or tautomers thereof, or pharmaceutically acceptable salts, hydrates or solvates thereof.

In another preferred embodiment, the pharmaceutical composition is used to treat a disease selected from the group consisting of cancer, infectious disease, and autoimmune disease.

In another preferred embodiment, the cancer is selected from the group consisting of pancreatic cancer, bladder cancer, colorectal cancer, breast cancer, prostate cancer, kidney cancer, hepatocellular carcinoma, lung cancer, ovary cancer, cervical cancer, stomach cancer, esophageal cancer, melanoma, neuroendocrine cancer, central nervous system cancer, brain cancer, bone cancer, soft tissue sarcoma, non-small cell lung cancer, small cell lung cancer or colon cancer, skin cancer, lung cancer, urinary system tumor, blood tumor, glioma, digestive system tumor, reproductive system tumor, lymphoma, nervous system tumor, brain tumor, and head and neck cancer.

In another preferred embodiment, the cancer is selected from the group consisting of acute lymphocytic leukemia (ALL), acute myeloid leukemia (AML), chronic lymphocytic leukemia (CLL), small lymphocytic lymphoma (SLL), myelodysplastic syndrome (MDS), myeloproliferative disorder (MPD), chronic myeloid leukemia (CML), multiple myeloma (MM), non-hodgkin lymphoma (NHL), mantle cell lymphoma (MCL), follicular lymphoma, Waldestrom macroglobulinemia (WM), T-cell lymphoma, B-cell lymphoma, and diffuse large B-cell lymphoma (DLBCL).

In another preferred embodiment, the infectious disease is selected from bacterial infection and viral infection.

In another preferred embodiment, the autoimmune disease is selected from the group consisting of organ-specific autoimmune disease and systemic autoimmune disease.

In another preferred embodiment, the organ-specific autoimmune diseases include chronic lymphocytic thyroiditis, hyperthyroidism, insulin-dependent diabetes mellitus, myasthenia gravis, ulcerative colitis, pernicious anemia with chronic atrophic gastritis, pulmonary hemorrhagic nephritic syndrome, primary biliary cirrhosis, multiple cerebral sclerosis, and acute idiopathic polyneuritis.

In another preferred embodiment, the systemic autoimmune diseases include rheumatoid arthritis, systemic lupus erythematosus, systemic vasculitis, scleroderma, pemphigus, dermatomyositis, mixed connective tissue disease, and autoimmune hemolytic anemia.

In another preferred embodiment, the pharmaceutical composition is also used to improve T cell function in a patient with chronic hepatitis B (CHB).

In another preferred embodiment, the inhibitor further comprises at least one therapeutic agent selected from the group consisting of nivolumab, pembrolizumab, atezolizumab and ipilimumab.

The sixth aspect of the present invention provides a method for regulating the interaction of PD-1/PD-L1 *in vivo,* which comprises the steps of: contacting the compound according to the first aspect of the present invention, or stereoisomers or tautomers thereof, or pharmaceutically acceptable salts, hydrates or solvates thereof with a PD-L1 protein.

### DETAILED DESCRIPTION OF THE INVENTION

After extensive and intensive research, the present inventors discovered a class of PD-1/PD-L1 interaction regulators with excellent regulatory effect. The present invention has been completed on this basis.

### Definitions

As used herein, the term "alkyl" includes straight or branched alkyl groups. For example, C₁-C₈ alkyl refers to straight or branched alkyls having from 1-8 carbon atoms, such as methyl, ethyl, propyl, isopropyl, butyl, isobutyl, tert-butyl, and the like.

As used herein, the term "alkenyl" includes straight or branched alkenyl groups. For example, C₂-C₆ alkenyl refers to straight or branched alkenyl groups having 2-6 carbon atoms, such as vinyl, allyl, 1-propenyl, isopropenyl, 1-butenyl, 2-butenyl, and the like.

As used herein, the term "alkynyl" includes straight or branched alkynyl groups. For example, "C₂-C₆ alkynyl" refers to straight or branched alkynyl group having 2-6 carbon atoms, such as ethynyl, propynyl, butynyl, and the like.

As used herein, the term "C₃-C₁₀ cycloalkyl" refers to cycloalkyl groups having 3 to 10 carbon atoms. It may be a monocyclic ring, such as cyclopropyl, cyclobutyl, cyclopentyl, cyclohexyl, and the like. It may also be in bicyclic form, such as bridged or spiro ring form.

As used herein, the term "C₁-C₈ alkylamino" refers to amine groups substituted with C₁-C₈ alkyl group, which may be mono- or di-substituted; for example, methylamino, ethylamino, propylamino, isopropylamine, butylamine, isobutylamine, tert-butylamine, dimethylamine, diethylamine, dipropylamine, diisopropylamine, dibutylamine, diisobutylamine, di-tert-butylamine, and the like.

As used herein, the term "C₁-C₈ alkoxy" refers to straight or branched alkoxy groups having 1-8 carbon atoms; for example, methoxy, ethoxy, propoxy, isopropoxy, butoxy, isobutoxy, tert-butoxy, and the like.

As used herein, the term "3-10 membered heterocycloalkyl having 1-3 heteroatoms selected from the group consisting of N, S and O" refers to a saturated or partially saturated cyclic group having 3-10 atoms, wherein 1-3 atoms are heteroatoms selected from the group consisting of N, S and O. It may be a monocyclic ring or in a bicyclic form, such as bridged or spiro ring form. Specific examples may be oxetane, azetidine, tetrahydro-2H-pyranyl, piperidinyl, tetrahydrofuranyl, morpholinyl, pyrrolidinyl, and the like.

As used herein, the term "C₆-C₁₀ aryl" refers to aryl groups having 6 to 10 carbon atoms, such as phenyl, naphthyl, and the like.

As used herein, the term "5-10 membered heteroaryl having 1-3 heteroatoms selected from the group consisting of N, S and O" refers to cyclic aromatic groups having 5-10 atoms, of which 1-3 atoms are selected from the group consisting of N, S and O. It may be a monocyclic ring or in a fused ring form. Specific examples may be pyridyl, pyridazinyl, pyrimidinyl, pyrazinyl, triazinyl, pyrrolyl, pyrazolyl, imidazolyl, (1,2,3)-triazolyl and (1,2,4)-triazolyl, tetrazyl, furyl, thienyl, isoxazolyl, thiazolyl, oxazolyl, and the like.

Unless otherwise specified as "substituted or unsubstituted", the group described in the present invention can be substituted by a substituent selected from the group consisting of halogen, nitrile, nitro, hydroxy, amino, C₁-C₆ alkyl-amine, C₁-C₆ alkyl, C₂-C₆ alkenyl, C₂-C₆ alkynyl, C₁-C₆ alkoxy, halogenated C₁-C₆ alkyl, halogenated C₂-C₆ alkenyl, halogenated C₂-C₆ alkynyl, halogenated C₁-C₆ alkoxy, allyl, benzyl, C₆-C₁₂ aryl, C₁-C₆ alkoxy-C₁-C₆ alkyl, C₁-C₆ alkoxy-carbonyl, phenoxycarbonyl, C₂-C₆ alkynyl-carbonyl, C₂-C₆ alkenyl-carbonyl, C₃-C₆ cycloalkyl-carbonyl, C₁-C₆ alkyl-sulfonyl, etc.

As used herein, "halogen" or "halogen atom" refers to F, Cl, Br, and I. More preferably, the halogen or halogen atom is selected from F, Cl and Br. "Halogenated" means substituted with an atom selected from F, Cl, Br, and I.

Unless otherwise specified, the structural formula described herein are intended to include all isomeric forms (such as enantiomeric, diastereomeric, and geometric isomers (or conformational isomers)): for example, R, S configuration of compounds with asymmetrical centers, (Z), (E) isomers of double bonds, etc. Therefore, the single stereochemical isomers or enantiomers, diastereomers or geometric isomers (or conformers) of the compounds of the invention, or mixtures thereof all fall within the scope of the invention.

As used herein, the term "tautomer" means that structural isomers having different energies can exceed the low energy barrier and thereby transform between each other. For example, proton tautomers (proton shift) include interconversion by proton transfer, such as 1H-carbazole and 2H-carbazole. Valence tautomers include interconversion through some bonding electron recombination.

As used herein, the term "solvate" refers to a complex formed by coordinating a compound of the invention with a solvent molecule in specific proportion.

As used herein, the term "hydrate" refers to a complex formed by coordinating a compound of the invention with water.

### Active ingredient

As used herein, "compounds of the present invention" refers to compounds of formula I, and also includes various crystalline forms, pharmaceutically acceptable salts, hydrates or solvates of the compounds of formula I.

Preferred compounds of the present invention include compounds 1-360 (including various Rand/or S-configuration stereoisomers, and/or E-/Z- cis-trans isomers of each compound).

In another preferred embodiment, the pharmaceutically acceptable salts include salts formed by combining with inorganic acids, organic acids, alkali metal ions, alkaline earth metal ions or organic bases capable of providing physiologically acceptable cations and ammonium salts.

In another preferred embodiment, the inorganic acids are selected from hydrochloric acid, hydrobromic acid, phosphoric acid or sulfuric acid; the organic acids are selected from methanesulfonic acid, p-toluenesulfonic acid, trifluoroacetic acid, medlaric acid, maleic acid, tartaric acid, fumaric acid, citric acid or lactic acid; the alkali metal ions are selected from lithium ion, sodium ion, potassium ion; the alkaline earth metal ions are selected from calcium ion, magnesium ion; and the organic bases capable of providing physiologically acceptable cations are selected from methylamine, dimethylamine, trimethylamine, piperidine, morpholine or tris(2-hydroxyethyl)amine.

All of these salts within the scope of the present invention can be prepared using conventional methods. During the preparation of the compounds of general formula I, solvates and salts thereof, polycrystalline or co-crystal may occur under different crystallization conditions.

### Preparation of compound I

In order to prepare the compounds described in general formula I of the present invention, based on the structure of general formula I, in the present invention, the preparation of general formula I can be obtained by the following synthesis routes. 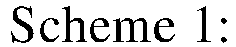 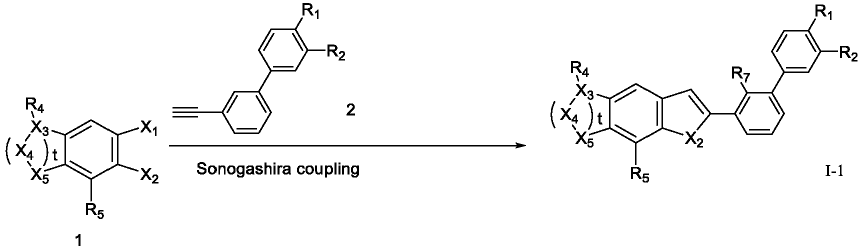
(a) providing the target product **1-1** by coupling reaction catalyzed by appropriate palladium catalyst by using intermediate **1** and **2** as raw materials;
the preparation method of intermediate **1** is as follows: 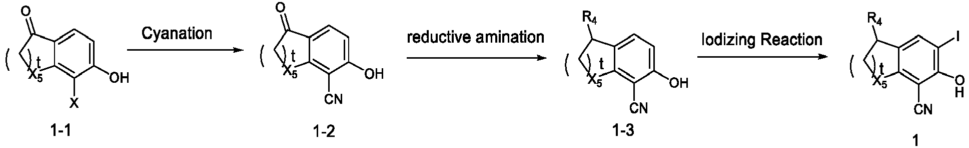
(a) providing intermediate **1-2** by coupling reaction with appropriate palladium catalyst and ligand using compound **1-1** (X is Cl, Br or I) as raw material;
(b) providing intermediate **1-3** by first condensation reaction with primary and secondary amines under the catalysis of appropriate Lewis acid using **1-2** as the raw material, then conducting reduction reaction by adding appropriate reducing agent; or obtaining the above by directly reductive amination reaction in the presence of appropriate reducing agent;
(c) providing intermediate **1** by halogenation reaction catalyzed by appropriate Lewis acid using **1-3** as raw material;
wherein, the preparation method of intermediate **1** is as follows: 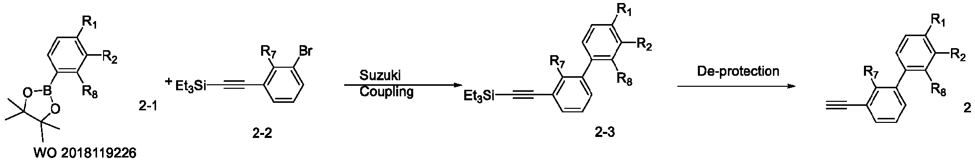
(a) providing intermediate **2-3** by coupling reaction (such as Suzuki, Buchwald, etc.) of compound **2-1** and **2-2** with appropriate palladium catalyst and ligand;
(b) providing intermediate **2** by removing silyl protecting group of **2-2** with appropriate agent.

In addition, the starting materials and intermediates in the above reactions are readily available, and each step of the reactions can be easily synthesized according to the known literature or by conventional methods in organic synthesis for those skilled in the art. The compound described in general formula I may be present as a solvate or non-solvate form, and different solvates may be obtained by crystallization with different solvents. 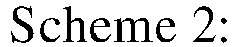 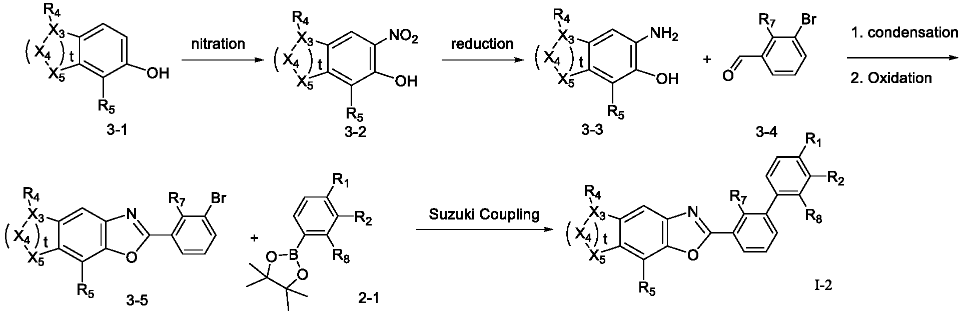
(a) providing intermediate **3-2** by using appropriate nitrating agent (such as concentrated sulfuric acid/NaNO₃, concentrated sulfuric acid/fuming nitric acid, etc.) to react with compound **3-1;**
(b) providing intermediate **3-3** by reducing **3-2** under appropriate reductive conditions (Pd-C/H₂; zinc powder/ammonium chloride; iron powder/acetic acid, etc.);
(c) providing intermediate **3-5** by condensing **3-3** and **3-4** with condensation agent, then oxidating with appropriate oxidant (such as DDQ);
(d) providing intermediate **1-2** by coupling **3-5** and **2-1** with appropriate catalyst and ligand.

In addition, the starting materials and intermediates in the above reactions are readily available, and each step of the reactions can be easily synthesized according to the reported literature or by conventional methods in organic synthesis for those skilled in the art. The compound described in general formula I may be present as a solvate or a non-solvate, and different solvates may be obtained by crystallization with different solvents.

### Pharmaceutical composition and administration method

Since the compounds of the present invention have excellent regulatory activity against PD-1/PD-L1 interaction, the compound of the present invention and various crystal forms thereof, pharmaceutically acceptable inorganic or organic salts, hydrates or solvates thereof, and pharmaceutical composition containing the compound according to the present invention as main active ingredient can be used to prevent and/or treat (stabilize, alleviate or cure) a disease associated with PD-1/PD-L1 interaction (eg, cancer, infectious disease, autoimmune disease).

The pharmaceutical composition of the invention comprises the compound of the present invention in a safe and effective dosage range and pharmaceutically acceptable excipients or carriers. Wherein the "safe and effective dosage" means that the amount of compound is sufficient to significantly ameliorate the condition without causing significant side effects. Generally, the pharmaceutical composition contains 1-2000 mg compounds of the invention per dose, preferably, 10-200mg compounds of the present invention per dose. Preferably, the "dose" is a capsule or tablet.

"Pharmaceutically acceptable carrier" means one or more compatible solids or liquid fillers, or gelatinous materials which are suitable for human use and should be of sufficient purity and sufficiently low toxicity. "Compatibility" means that each component in the composition can be admixed with the compounds of the present invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agent (such as sodium dodecyl sulfate), coloring agents, flavoring agents, stabilizers, antioxidants, preservatives, pyrogen-free water, etc.

There is no special limitation of administration mode for the compound or pharmaceutical compositions of the present invention, and the representative administration mode includes (but is not limited to): oral administration, parenteral (intravenous, intramuscular or subcutaneous) administration.

Solid dosage forms for oral administration include capsules, tablets, pills, powders and granules. In these solid dosage forms, the active compounds are mixed with at least one conventional inert excipient (or carrier), such as sodium citrate or CaHPO4, or mixed with any of the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrating agents such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, sodium lauryl sulfate, or the mixtures thereof. In capsules, tablets and pills, the dosage forms may also contain buffering agents.

The solid dosage forms such as tablets, sugar pills, capsules, pills and granules can be prepared by using coating and shell materials, such as enteric coatings and any other materials known in the art. They can contain an opaque agent. The release of the active compounds or compounds in the compositions can be released in a delayed mode in a given portion of the digestive tract. Examples of the embedding components include polymers and waxes. If necessary, the active compounds and one or more above excipients can form microcapsules.

Liquid dosage forms for oral administration include pharmaceutically acceptable emulsions, solutions, suspensions, syrups or tinctures. In addition to the active compounds, the liquid dosage forms may contain any conventional inert diluents known in the art such as water or other solvents, solubilizers and emulsifiers, for example, ethanol, isopropanol, ethyl carbonate, ethyl acetate, propylene glycol, 1,3-butanediol, dimethyl formamide, as well as oil, in particular, cottonseed oil, peanut oil, corn germ oil, olive oil, castor oil and sesame oil, or the combination thereof.

Besides these inert diluents, the composition may also contain additives such as wetting agents, emulsifiers, and suspending agent, sweetener, flavoring agents and perfume.

In addition to the active compounds, the suspension may contain suspending agent, for example, ethoxylated isooctadecanol, polyoxyethylene sorbitol and sorbitan esters, microcrystalline cellulose, methanol aluminum and agar, or a combination thereof.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols and any suitable mixtures thereof.

Compounds of the present invention can be administrated alone, or in combination with any other pharmaceutically acceptable compounds (such as other anticaner agents).

In the case of co-administration, the pharmaceutical composition can also include one or more (2, 3, 4, or more) other pharmaceutically acceptable compounds. One or more (2, 3, 4, or more) other pharmaceutically acceptable compounds may be used simultaneously, separately or sequentially with the compound of the present invention for the prevention and/or treatment of PD-1/PD-L1 interation related diseases.

When the pharmaceutical compositions are used, a safe and effective amount of compound of the present invention is applied to a mammal (such as human) in need thereof, wherein the dose of administration is a pharmaceutically effective dose. For a person weighed 60 kg, the daily dose is usually 1-2000 mg, preferably 20-500mg. Of course, the particular dose should also depend on various factors, such as the route of administration, patient healthy status, which are well within the skills of an experienced physician.

### The main advantages of the present invention include:

(1) The compounds of the present invention have high regulatory activity on PD-1/PD-L1 interaction, strong binding ability to PD-L1 protein, and the ability to relieve the inhibition of IFNy by PD-L1.
(2) The compounds of the present invention have better solubility; low toxicity to normal cells, so they can be administered to a subject in a larger dosage range.
(3) Compared with the compounds of the prior art, the compounds of the present invention have better solubility, so they have good druggability. Compared with the existing compounds, the compounds of the present invention show good bioavailability in *in vivo* experiments. In addition, compared with existing compounds, the compounds of the present invention can be easily made into pharmaceutically acceptable salts, thus facilitating further formulation.
*(4) In vivo* pharmacodynamic studies show that the compounds of the present invention can significantly inhibit the growth of subcutaneous tumors in terms of tumor volume and weight, and can significantly increase the number of lymphocytes in the blood and spleen of mice.

The present invention will be further illustrated below with reference to the specific examples. It should be understood that these examples are only to illustrate the invention but not to limit the scope of the invention. The experimental methods with no specific conditions described in the following examples are generally performed under the conventional conditions, or according to the manufacturer's instructions. Unless indicated otherwise, parts and percentage are calculated by weight.

The experimental materials and reagents used in the following examples can be commercially available unless otherwise specified.

### General Materials and Test Methods:

The instruments and raw materials involved in the examples are described as follows:
H NMR spectra were obtained by Bruker AV-400 (400MHz) NMR analysis.

Chemical shifts were recorded with tetramethylsilane as an internal standard and were expressed in ppm (CDCl₃: δ 7.26 ppm). The recorded data information was as follows: chemical shifts and their splitting and coupling constants (s: singlet; d: doublet; t: triplet; q: quartet; br: broad; m: multiplet).

Unless otherwise necessary, mass spectral data were analyzed by using LC/MS spectrometer of Finnigan LCQ Advantage, and all reactions were conducted under dry argon protected anhydrous and oxygen-free conditions. The solid metal organic compounds were stored in an argon-protected dry box.

Tetrahydrofuran and ether were obtained by distillation, in which sodium metal and benzophenone were added. Dichloromethane, pentane and hexane were treated with calcium hydride.

The special raw materials and intermediates involved in the present invention are customized and provided by Tianjin Longwood Pharmaceutical Co., Ltd., etc., and all other chemical reagents are purchased from reagent suppliers such as Shanghai Chemical Reagent Company, Aldrich, and Acros, etc.. If the intermediates or products required for the reaction in the synthesis process are not enough for the next step test, the synthesis is repeated several times to sufficient amount.

Unless otherwise specified, the raw materials and reagents involved in the present invention can be commercially available or can be purchased through customized processing.

The compounds of the present invention may contain one or more asymmetric centers, and thus the series of compounds may be in racemic or single enantiomeric form. The compounds prepared in the present invention are heterocyclic compounds with a purity of more than 95%, and the structural characterization of each final product is determined by MS or/and hydrogen spectral nuclear magnetic resonance (¹H NMR) analysis, respectively. The following examples illustrate the synthesis of various compounds and intermediates of the present invention.

### Example 1 Synthesis of compound 001

### Step 1-1:

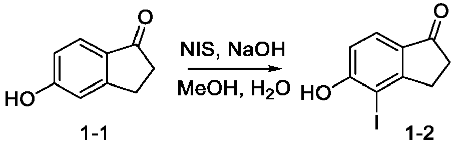

Raw material 1-1 (100 g, 1.0 eq) and sodium hydroxide (27 g, 1.0 eq) were added into water (500 ml) and methanol (2000 ml), the mixture obtained was brown and slightly muddy, and cooled down to about -25 °C in the internal bath. NIS (151 g, 1.0 eq) was added at one time, then naturally warmed to react, the amount of solid was increased, then gradually dissolved until the solution turned to dark brown and clear. The reaction was conducted for 2 hours and tracked by TLC and LCMS. 1000ml of water was added, and 2M hydrochloric acid (400ml) was added. The system was weakly acidic, filtered, and rinsed with MTBE twice to obtain 161g of solid and 87% of yield. MS-APCI: 275 [M+H]⁺.

### Step 1-2:

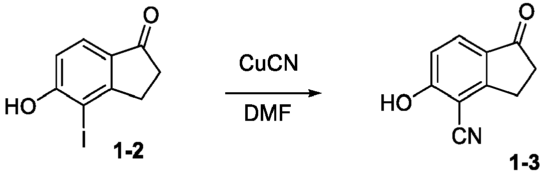

DMF (1000ml, 2BV) was added into the reaction bottle, tunder the protection of N₂, raw material 1-2 (500g, 1.0eq) and cuprous cyanide (172g, 1.05eq) were added. The atmosphere was replaced with nitrogen and warmed to to 115 °C in the internal bath. The reaction was conducted for 6 hours with LCMS tracking. Post treatment: After diluted with THF, the mixture was filtered, and the filtrate was concentrated. Sodium hydroxide aqueous solution (1.1eq, 6BV) was added at room temperature to adjust pH=8~9; the mixture was filtered, the filtrate was adjusted to pH=7 with 2M sulfuric acid, ferrous sulfate and activated carbon were added, stirred at room temperature for 1 hour. The mixture was filtered, the filtrate was adjusted to pH=2~3 with 2M sulfuric acid, and the solid was filtered and pulped with isopropanol (200ml) for 30 minutes, filtered, and dried to obtain 211g of brown solid. MS-APCI: 174 [M+H]⁺.

### Steps 1-3:

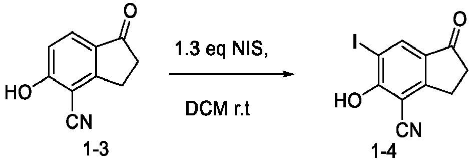

4.5 g of compound 1-3 was dissolved in 200 ml of DCM (in a 500 ml single necked flask), 6.3 g of NIS was added, and was protected with argon. The reaction was conducted overnight at room temperature. TLC showed that 1-2 was reacted completely. 100 mL of 2M HCl was added to the reaction solution, and the reaction solution was separated and dried. The mixture was evaporated by rotary evaporation and purified by a column (Hep DCM 1:1, then DCM) to obtain 6 g of the product as light yellow solid. MS-APCI: 300 [M+H]⁺.

### Steps 1-4:

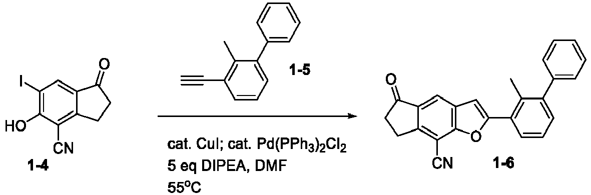

945 mg of compound 1-4, 636 mg of compound 1-5, 25 mg of CuI and 25 mg of Pd(PPh₃)₂Cl₂ were added in a 50 ml three necked flask under the protection of argon, 20 ml of DMF and 1.2 ml of DIPEA were added, and the reaction was conducted at 55°C for 3 hours. TLC showed that the raw material disappeared. The reaction solution was added to 300ml of ice water, extracted twice with 300ml of ethyl acetate, washed once with 2M NaOH, dried and evaporated by rotary evaporation. The mixture was purified by a column (DCM-MeOH 10:1) to obtain 120 mg of light yellow solid. MS-APCI: 364 [M+H]⁺.

### Steps 1-5:

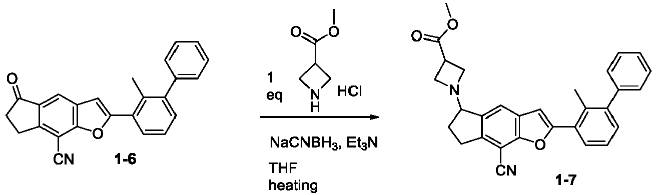

215 mg of compound 1-6, 248 mg of methyl azocyclobutane-3-carboxylate hydrochloride, 125 mg of Na(CN)BH₃ were dissolved in 10 ml of THF under the protection of argon. 167 mg of triethylamine was added, and the reaction was conducted for 24 hours at 70°C. The reaction solution was added to 100ml of ice water, extracted twice with 100ml of EA and dried. The mixture was purified by a column (DCM-MeOH 100, 50:1, 10:1: 5:1) to obtain 53 mg of light yellow solid 1-7, MS-APCI: 463 [M+H]⁺.

### Steps 1-6:

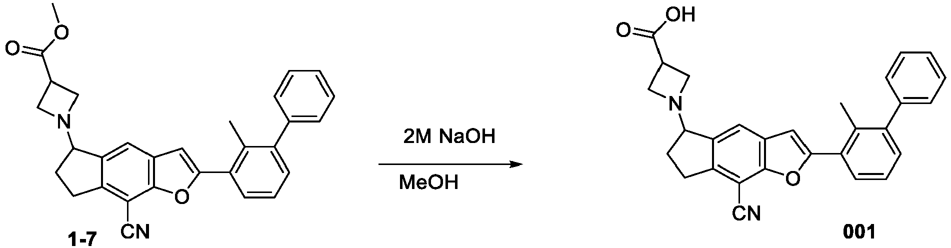

53 mg of raw material 1-7 was dissolved in 5 ml of MeOH, 2 ml of 2M NaOH was added, and the mixture was reacted at 26 °C for 5 hours. The reaction solution was evaporated by rotary evaporation at 40 °C, 50 ml of water was added to the residual solid. 2M HCl was added to the solution to adjust pH 7. The solid was filtered and washed with water and THF, dried, and further purified to obtain 5 mg of yellow solid. MS-APCI: 449 [M+H]⁺.

The compounds in the following table were also synthesized by the synthesis method of compound 001:

| Number | Compound structure | LCMS [M+H]⁺ |
|---|---|---|
| 001 | 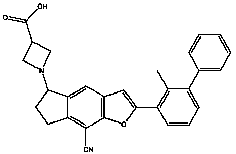 | 449.2 |
| 003 | 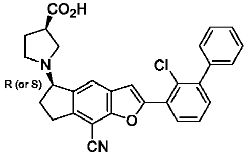 | 483.2 |
| 004 | 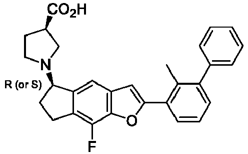 | 456.2 |
| 005 | 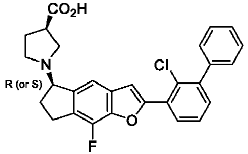 | 476.2 |
| 006 | 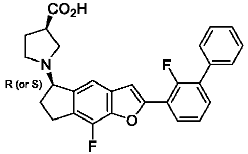 | 460.2 |
| 007 | 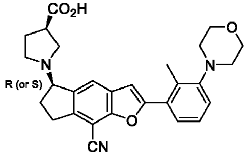 | 472.2 |
| 008 | 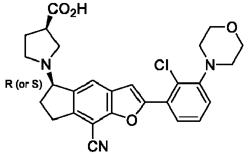 | 492.2 |
| 009 | 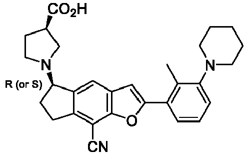 | 470.2 |
| 010 | 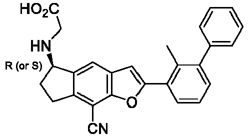 | 423.2 |
| 011 | 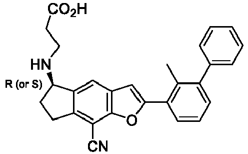 | 437.2 |
| 012 | 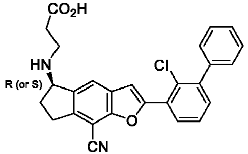 | 457.2 |
| 013 | 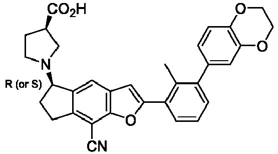 | 521.2 |
| 014 | 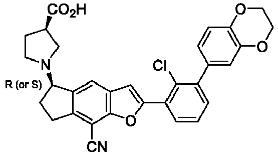 | 541.2 |
| 015 | 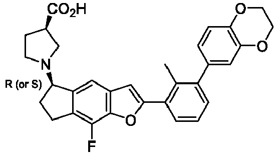 | 514.2 |
| 016 | 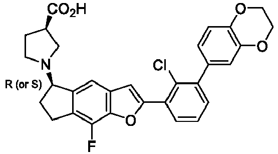 | 534.2 |
| 017 | 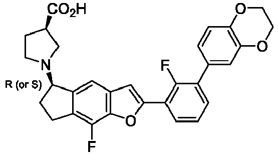 | 518.2 |
| 018 | 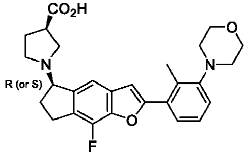 | 465.2 |
| 019 | 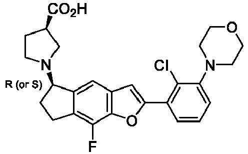 | 485.2 |
| 020 | 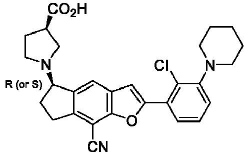 | 490.2 |
| 021 | 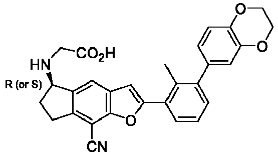 | 481.2 |
| 022 | 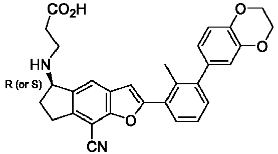 | 495.2 |
| 023 | 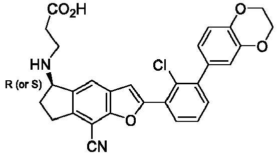 | 515.2 |
| 024 | 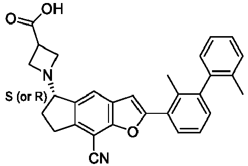 | 463.2 |
| 093 | 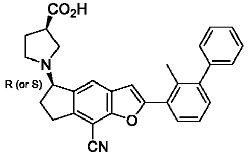 | 463.2 |
| 115 | 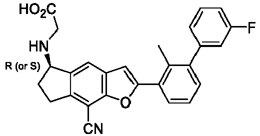 | 441.2 |
| 116 | 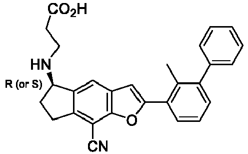 | 437.2 |
| 117 | 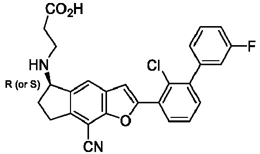 | 475.2 |
| 118 | 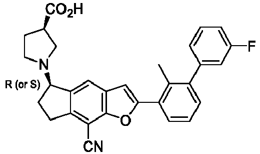 | 481.2 |
| 119 | 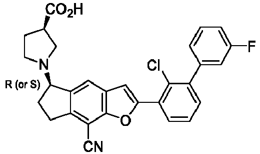 | 501.2 |
| 120 | 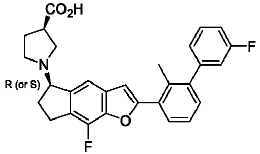 | 474.2 |
| 121 | 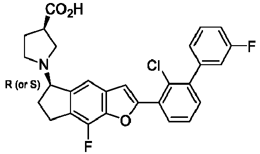 | 494.2 |
| 122 | 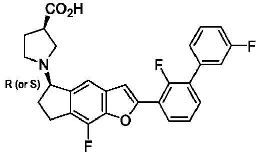 | 478.2 |
| 123 | 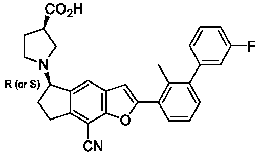 | 481.2 |
| 124 | 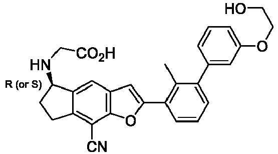 | 482.2 |
| 125 | 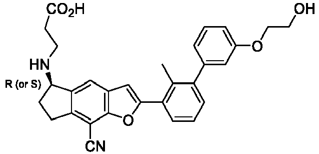 | 497.2 |
| 126 | 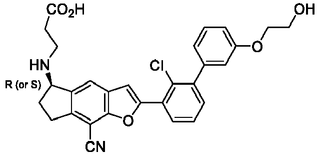 | 517.2 |

### Example 2 Synthesis of compound 026

### Step 2-1:

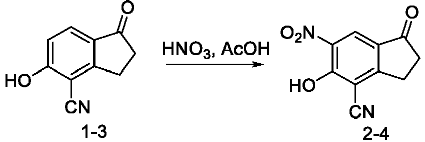

Raw material 1-3 (110g, 1.0eq) was added to acetic acid (660ml, 6BV), the resulting mixture was turbid, and warmed to 40 °C in the internal bath. Fuming nitric acid (84g, 2.1eq) was slowly added, the resulting mixture was turbid, and then the temperature was kept to react for about 1 hour. LCMS tracking showed that 3.5% of the raw materials remained. Post treatment: the reaction solution was cooled to 10 °C, stirred for 30 minutes, filtered, rinsed with HEP, dried to obtain 117g of solid with yield of 84%, purity 88%, 8% of raw material was added into THF (600ml) and pulped for 1 hour in an external bath at 50 °C, filtered at room temperature to obtain 106g of solid, yield 76%. MS-APCI: 219 [M+H]⁺.

### Step 2-2:

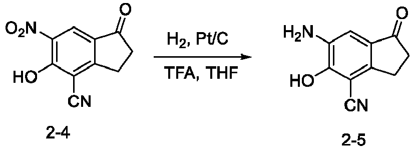

2-4 (20g, 1.0eq), TFA (12g, 1.1eq) were added into THF (400ml, 20BV), the resulting mixture was turbid. 5% platinum-carbon (10-20%) was added, replaced by hydrogen, and the reaction was conducted at room temperature and tracked by LCMS (reaction speed was affected by many factors). Post-treatment: the reaction solution was filtrated, washed with 3BVTHF, then used the next step directly. MS-APCI: 189 [M+H]⁺.

### Step 2-3:

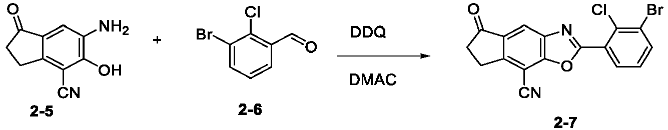

2.02g of compound 2-3 was dissolved in 50ml of DMAC, 1.98g of 2-4 was added, and the mixture was reacted at room temperature for 3h under the protection of argon. 3g of DDQ was added and the mixture was reacted overnight at room temperature. The reaction solution was poured into 150ml of water, extracted twice by DCM (100ml), and dried. The mixture was evaporated by rotary evaporation and purified by a column (DCM-MeOH 100, 50:1, 30:1, 20:1, 10:1, 5:1) to obtain 100 mg of product as a light yellow solid. MS-APCI: 387 [M+H]⁺.

### Step 2-4:

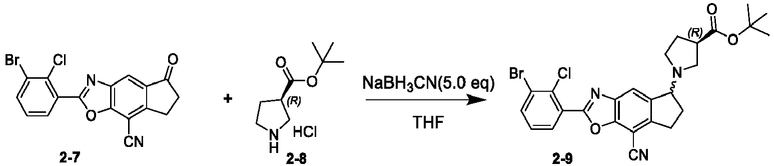

Intermediate 2-7 (20.7 g, 3.3eq) was dissolved in THF (143 mL, 10V/W), intermediate 2-8 (14.3 g, 1.0 eq) was added, NaBH₃CN (5.0 eq, 11.6 g) was added, and replaced with nitrogen. The mixture was stirred in 70 °C in an oil bath overnight. LC-MS detection showed that 15% of 2-7 was left, then the reaction was stopped and cooled to room temperature. The mixture was combined with another batch (1g), water was added and extracted twice with EA. The combined organic phases was washed with brine, dried and concentrated. The mixture was purified by column (MeOH in DCM (2%)) to obtain 17.2 g of product and 5.0 g of off-white solid at the intersection. The mixture was pulped with DCM: Hep 1:1 100mL and 20mL, respectively, stirred at room temperature for 10min, filtered, and rinsed with Hep. Filter cakes were collected and dried to obtain 10.3g and 1.0g respectively, where no obvious impurities were detected by LCMS, then combined to obtain 11.1g of off-white solid with HPLC of 97.9% and yield of 51.8%. After chiral separation, peak 1 4.48 g and peak 2 5.12 g were obtained.

### Step 2-5:

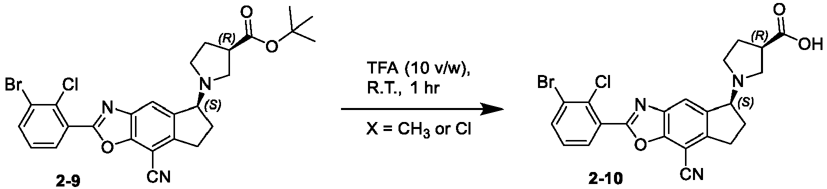

tert-Butyl ester 2-9 (1 g, 1.0 eq) was added in TFA (10 v/w) and the mixture was stirred for 1 hour at room temperature. LCMS detected that the reaction was completed. Then the mixture was directly concentrated to obtain yellow to brown oil. Water (10 v/w) was added to the concentrated residue, viscous semioily and semisolid substance was precipitated, saturated NaHCO₃ solution was used to adjust pH to 6-7, and a large number of viscous semioily and semisolid substance were gathered together. The mixture was coarse filtered once, filter cake (or paste) was collected, water (20 v/w) was added, ultrasonicated for 5-10 minutes, and the viscous substance gradually solidified and partially dispersed. After stirred at room temperature for 2 hours, the substance was almost dispersed, filtered, washed, the filter cake was collected, and dried to obtain 500 mg of product, MS-APCI: 486 [M+H]⁺.

### Step 2-6:

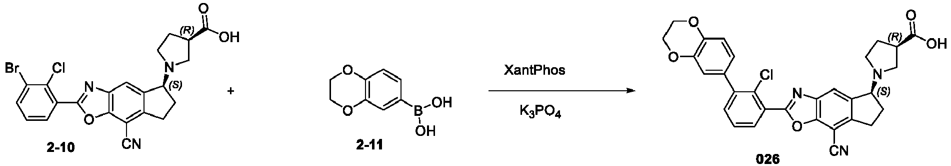

Borate raw material 2-11 (commercial, 37 mg), 2-10 (100 mg), potassium phosphate (88 mg) and RuPhos-Pd-G3 (5 mg) were added into a single ended flask containing 1,4-dioxane/H₂O (4:1, 5mL) successively under the protection with nitrogen, the mixture was stirred in an oil bath at 90 °C for 1 hour under the detection of LC-MS. After the reaction was completed, the solution was filtered, evaporated by rotary evaporation, and prepared by Prep-HPLC to obtain 10 mg of compound 026 (white solid), ESI (APCI): 542 [M+H]⁺.

1H NMR (400 MHz, DMSO-d6) δ12.94 (s, 1H), 10.34 (s, 1H), 8.47 (s, 1H), 8.08 (dd, J = 6.9, 2.6 Hz, 1H), 7.77 - 7.39 (m, 2H), 7.10 - 6.64 (m, 3H), 5.12 (s, 1H), 4.28 (s, 4H), 3.67 - 3.4 (m, 3H), 3.24 - 3.15 (m, 3H), 2.37 - 2.10 (m, 2H), 2.00 - 1.93 (m, 1H).

The compounds in the following table were also synthesized by the synthesis method of compound 002:

| Number | Compound structure | LCMS [M+H]⁺ |
|---|---|---|
| 026 | 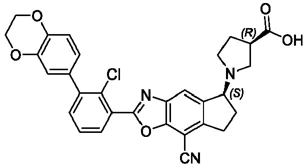 | 542.2 |
| 057 | 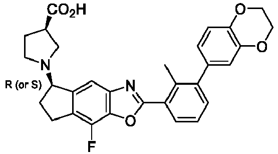 | 515.2 |
| 052 | 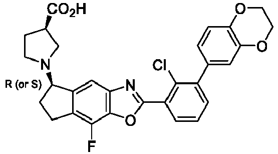 | 535.2 |
| 053 | 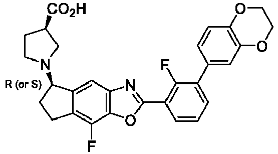 | 519.2 |
| 054 | 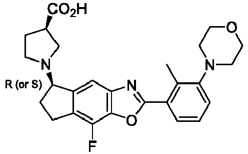 | 466.2 |
| 055 | 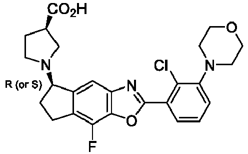 | 486.2 |
| 056 | 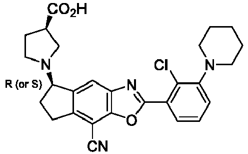 | 491.2 |
| 027 | 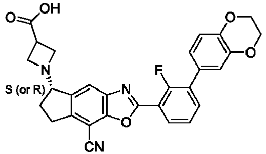 | 512.2 |
| 028 | 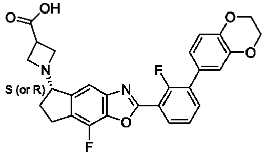 | 505.2 |
| 029 | 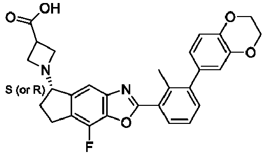 | 501.2 |
| 030 | 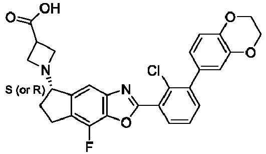 | 521.2 |
| 031 | 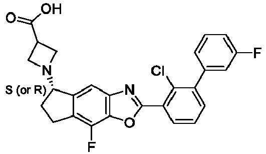 | 481.2 |
| 044 | 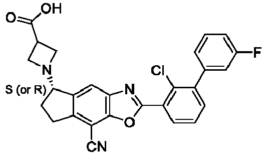 | 488.2 |
| 045 | 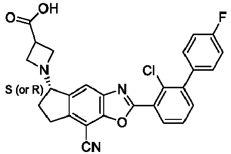 | 488.2 |
| 046 | 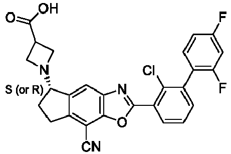 | 506.2 |
| 047 | 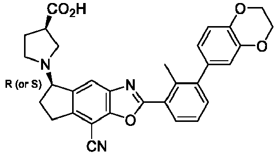 | 522.2 |
| 048 | 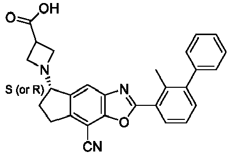 | 450.2 |
| 049 | 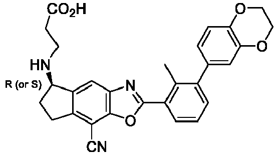 | 496.2 |
| 050 | 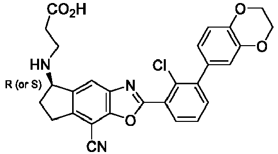 | 516.2 |
| 051 | 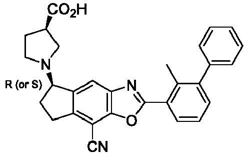 | 464.2 |
| 058 | 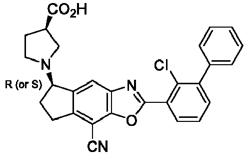 | 484.2 |
| 059 | 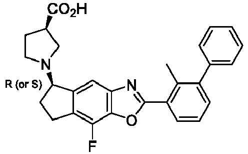 | 457.2 |
| 060 | 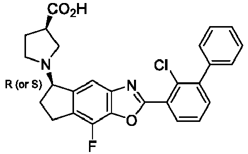 | 477.2 |
| 062 | 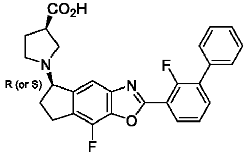 | 461.2 |
| 063 | 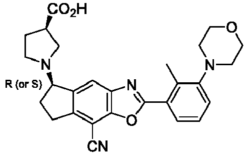 | 473.2 |
| 064 | 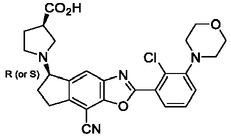 | 493.2 |
| 065 | 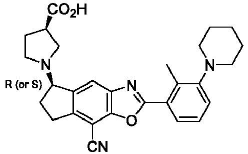 | 471.2 |
| 066 | 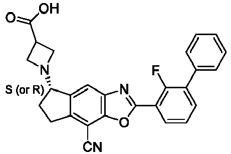 | 454.2 |
| 067 | 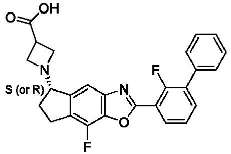 | 447.2 |
| 068 | 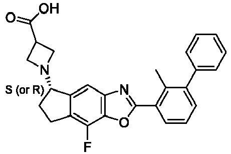 | 443.2 |
| 069 | 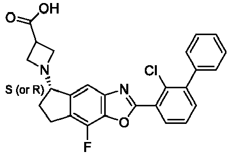 | 463.2 |
| 070 | 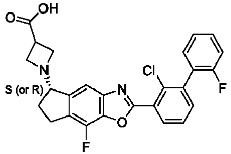 | 481.2 |
| 071 | 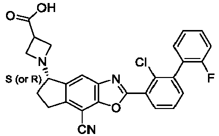 | 488.2 |
| 072 | 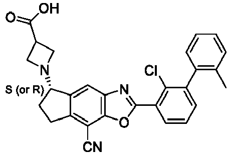 | 484.2 |
| 073 | 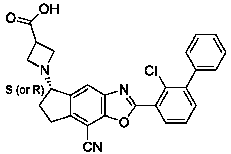 | 470.2 |
| 074 | 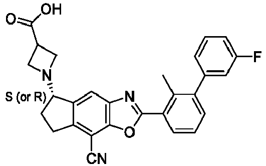 | 468.2 |
| 075 | 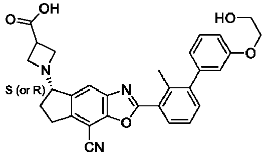 | 510.2 |
| 076 | 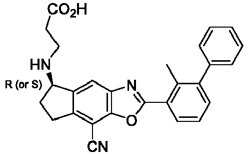 | 438.2 |
| 077 | 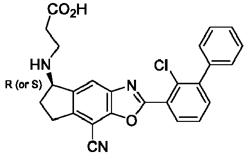 | 458.2 |
| 078 | 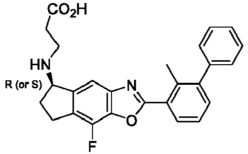 | 431.2 |
| 079 | 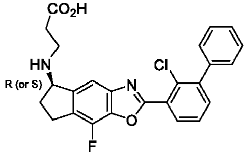 | 451.2 |
| 080 | 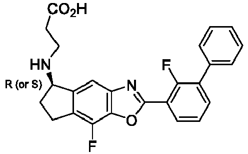 | 435.2 |
| 081 | 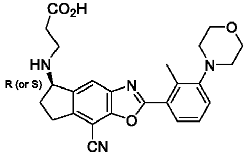 | 447.2 |
| 082 | 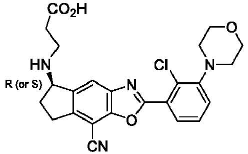 | 467.2 |
| 083 | 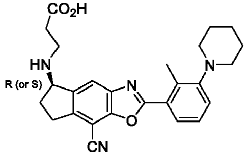 | 445.2 |
| 084 | 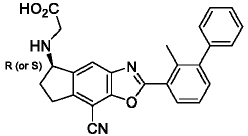 | 424.2 |
| 085 | 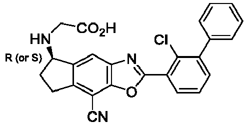 | 444.2 |
| 086 | 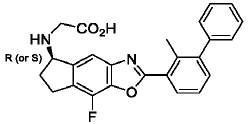 | 417.2 |
| 087 | 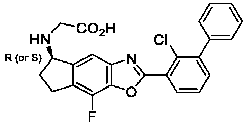 | 437.2 |
| 088 | 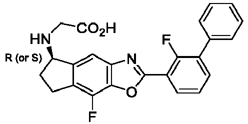 | 421.2 |
| 089 | 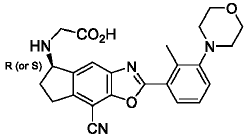 | 433.2 |
| 090 | 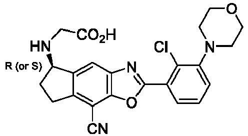 | 453.2 |
| 091 | 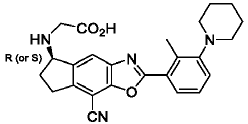 | 431.2 |
| 092 | 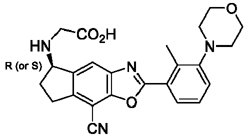 | 433.2 |
| 105 | 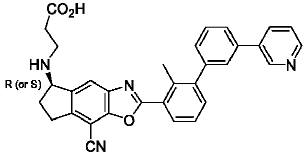 | 515.2 |
| 106 | 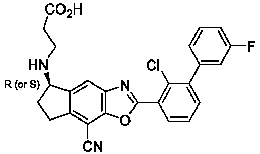 | 476.2 |
| 107 | 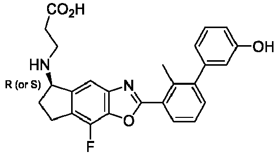 | 445.2 |
| 108 | 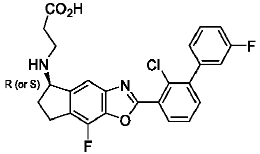 | 469.2 |
| 109 | 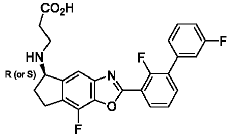 | 453.2 |
| 110 | 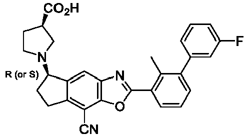 | 482.2 |
| 111 | 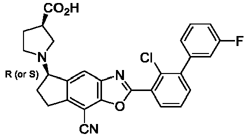 | 502.2 |
| 112 | 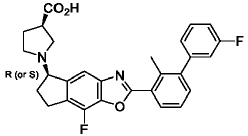 | 475.2 |
| 113 | 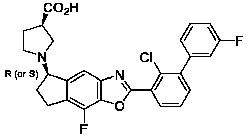 | 495.2 |
| 114 | 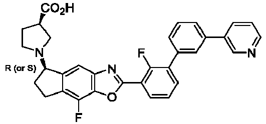 | 538.2 |
| 127 | 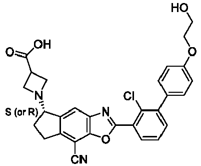 | 530.2 |
| 128 | 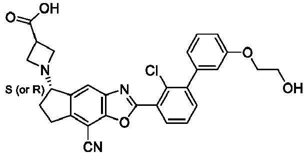 | 530.2 |
| 129 | 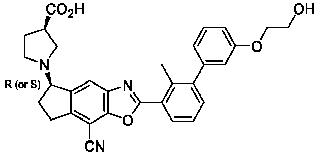 | 524.2 |
| 130 | 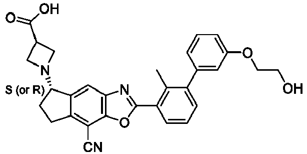 | 510.2 |
| 131 | 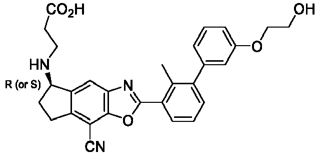 | 498.2 |
| 132 | 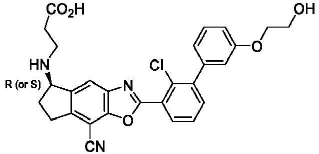 | 518.2 |

### Example 3 Synthesis of compound 025

### Step 3-1:

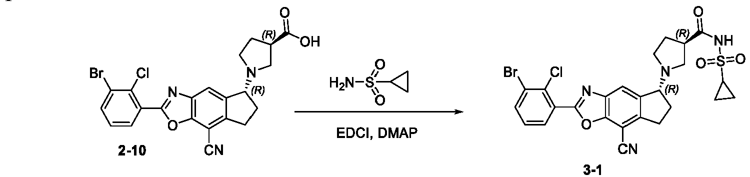

Compound 3-1 (1125 mg, 2.32 mmol), cyclopropylsulfonamide (310 mg, 2.56 mmol), DMAP (567 mg, 4.64 mmol) and EDCI (889 mg, 4.64 mmol) were added into a single ended flask containing DCM (15 mL) successively. The mixture was stirred at room temperature for 1 hour and detected by TLC (since UV signal was too weak, the plate was spotted after washed with 0.5 M diluted hydrochloric acid). After the reaction was completed, the reaction solution was washed with 0.5 M diluted hydrochloric acid and brine, dried over anhydrous sodium sulfate, and purified by column chromatography to obtain 548 mg of white solid. ESI (APCI): 589 [M-H]⁻.

### Step 3-2:

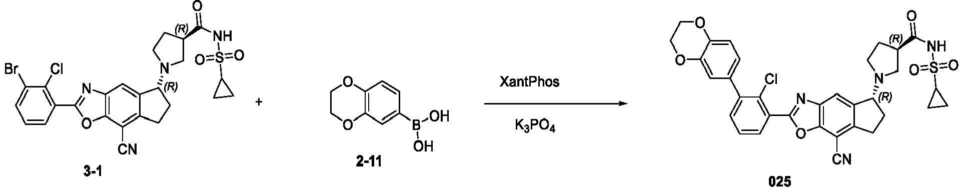

Borate raw material 2-11 (commercial, 28 mg), 2-10 (90 mg), potassium phosphate (88 mg) and XantPhos-PdCl₂ (13 mg) were added into a single necked flask containing 1,4-dioxane/H₂O (4:1, 5 mL) successively under the protection of nitrogen. The mixture was stirred in an oil bath at 90 °C for 2 hours, and detected by LC-MS. After the reaction was completed, the solution was filtered, evaporated by rotary evaporation, and prepared by Prep-HPLC to obtain 10 mg of compound 025 (white solid), ESI (APCI): 645 [M+H]⁺.

1H NMR (400 MHz, DMSO-d₆) δ 8.50 (s, 1H), 8.11 (dd, J = 6.8, 2.6 Hz, 1H), 7.72 - 7.61 (m, 2H), 6.99 (dd, J = 5.2, 3.1 Hz, 2H), 6.94 (dd, J = 8.3, 2.1 Hz, 1H), 5.17 (d, J = 7.0 Hz, 1H), 4.31 (s, 4H), 3.49 (d, J = 8.2 Hz, 2H), 3.32 (s, 2H), 3.24 (ddd, J = 17.2, 8.6, 3.1 Hz, 2H), 2.96 (d, J = 12.8, 6.6, 5.9 Hz, 2H), 2.69 - 2.54 (m, 2H).

| Number | Compound structure | LCMS [M+H]⁺ |
|---|---|---|
| 025 | 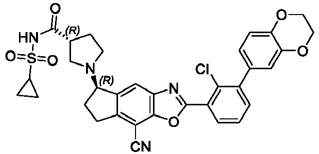 | 645.2 |
| 094 | 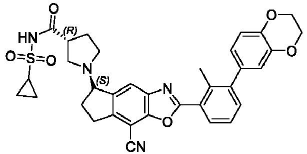 | 625.2 |
| 095 | 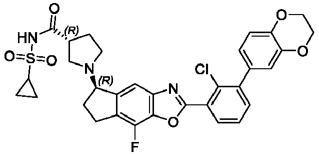 | 638.2 |
| 096 | 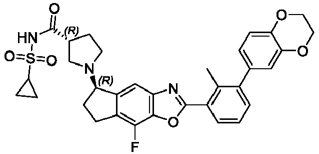 | 618.2 |
| 097 | 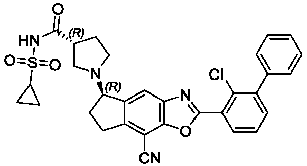 | 587.2 |
| 098 | 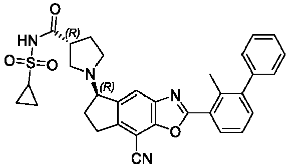 | 567.2 |
| 099 | 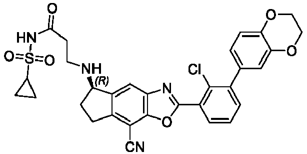 | 619.2 |
| 100 | 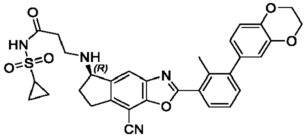 | 599.2 |
| 101 | 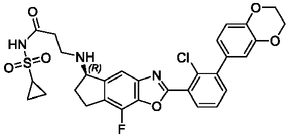 | 612.2 |
| 102 | 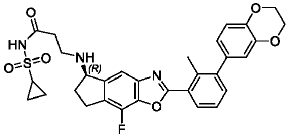 | 592.2 |
| 103 | 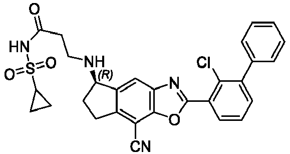 | 561.2 |
| 104 | 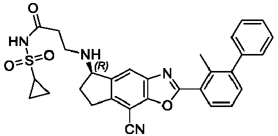 | 541.2 |
| 133 | 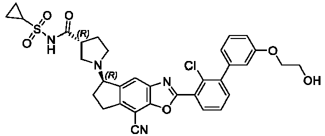 | 647.2 |
| 134 | 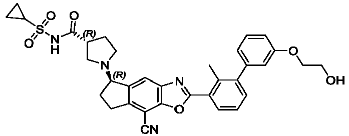 | 627.2 |
| 135 | 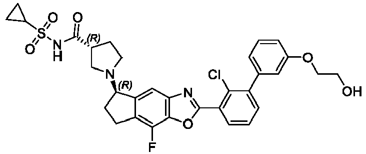 | 640.2 |
| 136 | 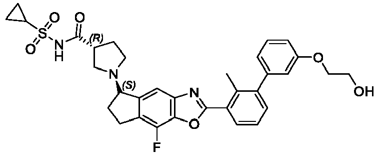 | 620.2 |
| 137 | 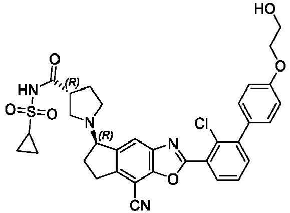 | 647.2 |

### Biological Test

### Example A: PD-1/PD-L1 Homogeneous Time-Resolved Fluorescence (HTRF) Binding Assay

Assays were performed in standard black 384-well polystyrene plates and a final volume was 20 µL. The inhibitor was first serially diluted with DMSO and added to the wells of the plate, followed by the addition of other reaction components. The final concentration of DMSO in the assay was 1%. Assays were performed at 25°C in PBS buffer (pH 7.4) containing 0.05% Tween-20 and 0.1% BSA. Recombinant human PD-L1 protein (19-238) with a His-tagged at the C-terminus was purchased from AcroBiosystems (PD1-H5229). Recombinant human PD-1 protein (25-167) with an Fc tag at the C-terminus was also purchased from AcroBiosystems (PD1-H5257). The PD-L1 and PD-1 proteins were diluted in assay buffer and then 0.1 µl of the solution was extracted and added to the wells of the plate. Plates were centrifuged and proteins and inhibitors were preincubated for 40 minutes. After incubation, 0.1 µl of HTRF detection buffer containing europium blocking labeled anti-human IgG (PerkinElmer-AD0212), Fc-specific and anti-His SureLight^{®}-Allophycocyanin (APC, PerkinElmer-AD0059H) conjugated antibodies was added. After centrifugation, the plates were incubated at 25°C for 60 minutes. Data was read in a PHERAstar FS plate reader (665nm/620nm ratio). Final concentrations in the assay were ~3 nM of PD1, 10 nM of PD-L1, 1 nM of europium anti-human IgG, and 20 nM of anti-His-allophycocyanin. Activity data were fitted using GraphPad Prism 5.0 software to derive IC₅₀ values for inhibitors.

The IC₅₀ values of the compounds exemplified in the examples are expressed in the following manner: IC₅₀: +: ≤100 nM; ++: 100 nM~1000 nM; +++: >1000 nM. The data of the example compounds obtained using the PD-1/PD-L1 homogeneous time-resolved fluorescence (HTRF) binding assay described in Example A were provided in Table 1.

| Compound | IC₅₀ |
|---|---|
| 013 | + |
| 025 | + |
| 026 | + |
| 047 | + |
| 049 | + |
| 050 | + |
| 005 | + |

All documents mentioned herein are incorporated by reference in the present invention as if each document was individually incorporated by reference. In addition, it should be understood that after reading the above teaching content of the present invention, those skilled in the art can make various changes or modifications to the present invention, and these equivalent forms also fall within the scope defined by the appended claims of the present application.

## Claims

1. A compound shown in Formula I below, or optical isomers, hydrates, solvates thereof, or pharmaceutically acceptable salts thereof: 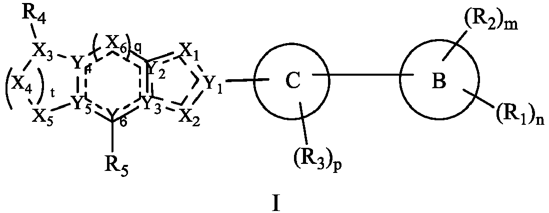
wherein, n is 0, 1, 2, or 3;
m, p, q, and t are each independently selected from 0, 1, 2, 3, or 4;
X₁, X₂, X₃, X₄, X₅ and X₆ are each independently selected from the group consisting of N, O, S, SO, SO₂, C(R)₂, or NR;
Y₁, Y₂, Y₃, Y₄, Y₅ and Y₆ are each independently selected from the group consisting of N, CH, or C; 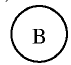
is selected from the group consisting of substituted or unsubstituted C1-C15 aryl, or substituted or unsubstituted 4-12 membered (preferably 5-7 membered) heteroaryl with 1-4 heteroatoms, substituted or unsubstituted 4-12 membered heterocyclyl, and substituted or unsubstituted 4-12 membered C5-C12 cycloalkyl; 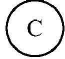
is selected from the group consisting of substituted or unsubstituted C1-C15 arylene, or substituted or unsubstituted 4-12 membered (preferably 5-7 membered) heteroarylene with 1-4 heteroatoms, substituted or unsubstituted 4-12 membered heterocyclylene, and substituted or unsubstituted 4-12 membered C5-C12 cycloalkylene;
R, R₁, R₂, R₃, R₄ and R₅ are each independently selected from the group consisting of H, - CN, trifluoromethyl, sulfonamido, nitro, hydroxyl, halogen, -S-R₈, -S(O)-R₈, -S(O)₂-R₈, substituted or unsubstituted C1-C10 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl, oxo (i.e.=O), =NRε, -CN, hydroxyl, NR_{d}Rₑ (e.g. amino), substituted or unsubstituted C1-C6 amino, substituted or unsubstituted -(C1-C6 alkylene)-NH-(C1-C6 alkylene), carboxyl, substituted or unsubstituted C6-C10 aryl, substituted or unsubstituted 5-12 membered heteroaryl with 1-3 heteroatoms, substituted or unsubstituted 3-12 membered heterocyclyl with 1-4 heteroatoms, substituted or unsubstituted 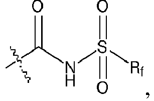 substituted or unsubstituted 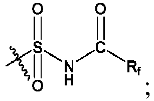 or -(L₁ₐ)ᵣ-(L₂ₐ)ₛ-(L₃ₐ)ₛ-; -C₀₋₈-O-R₈, -C₀₋₈-C(O)OR₈, -C₀₋₈-OC(O)OR₈, -C₀₋₈-NR₈R₉, -C₀₋₈-N(R₈)C(O)R₉, -C₀₋₈-C(O)NR₈R₉;
or R₁ and R₂ together with the adjacent ring atom form a 5, 6 or 7 membered carbon ring or a 5, 6 or 7 membered heterocycle, wherein the heterocycle contains 1, 2 or 3 heteroatoms selected from N, S or O; the carbon ring or heterocycle can be an aromatic ring conjugated with the Bring, or a non-aromatic ring containing unsaturated bond, or a saturated ring;
R₈ and R₉ are each independently selected from the group consisting of H, hydroxyl, substituted or unsubstituted C1-C10 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl, oxo (i.e.=O), =NRε, -CN, hydroxyl, NR_{d}Rₑ (e.g. amino), substituted or unsubstituted C1-C6 amino, substituted or unsubstituted -(C1-C6 alkylene)-NH-(C1-C6 alkylene), carboxyl, substituted or unsubstituted C6-C10 aryl, substituted or unsubstituted 5-12 membered heteroaryl with 1-3 heteroatoms, substituted or unsubstituted 3-12 membered heterocyclyl with 1-4 heteroatoms, substituted or unsubstituted 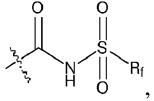 substituted or unsubstituted 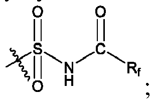 or -(L₁ₐ)ᵣ-(L₂ₐ)ₛ-(L₃ₐ)ₛ-;
each L₁ₐ is independently selected from the group consisting of chemical bond, substituted or unsubstituted C₁-C₇ alkylene, substituted or unsubstituted C2-C4 alkenylene, substituted or unsubstituted C2-C4 alkynylene, -S-, -O-, substituted or unsubstituted -NH-, -S(O)-, -S(O)₂-;
L₂ₐ is selected from the group consisting of substituted or unsubstituted C6-C12 arylene, substituted or unsubstituted 5-12 membered heteroarylene with 1-3 heteroatoms, substituted or unsubstituted C3-C8 cycloalkylene, substituted or unsubstituted 5-10 membered heterocyclylene with 1-3 heteroatoms;
L₃ₐ is selected from the group consisting of H, substituted or unsubstituted C1-C10 alkyl, C1-C10 aryl, -CN, hydroxyl, amino, carboxyl, -CO-NH-SO₂-R_{g}, -NH-SO₂-R_{g}, -SO₂-NH-CO-R_{g};
r is 1, 2, 3, 4, 5, or 6;
s is 0, 1, or 2, respectively;
R_{d}, Rₑ and R_{g} are each independently selected from the group consisting of H, substituted or unsubstituted C₁-C₆ alkyl, substituted or unsubstituted C₃-C₁₀ cycloalkyl, substituted or unsubstituted C₆-C₁₀ aryl; or R_{d} and Rₑ together form a substituted or unsubstituted 5-10 membered heterocyclyl with 1-3 heteroatoms selected from N, S and O;
R_{f} is selected from the group consisting of H, substituted or unsubstituted C₁-C₆ alkyl, substituted or unsubstituted C₆-C₁₀ aryl, substituted or unsubstituted C₆-C₁₀ heteroaryl, cyano, - C(=O)-NR_{d}Rₑ, -C(=O)-substituted or unsubstituted C₁-C₆ alkoxy, -C(=O)-substituted or unsubstituted C₁-C₆ alkyl, -C(=O)-substituted or unsubstituted C₃-C₁₀ cycloalkyl, -C(=O)-substituted or unsubstituted C₂-C₆ alkenyl, -C(=O)-substituted or unsubstituted C₂-C₆ alkynyl;
unless otherwise specified, the "substituted" means being substituted by one or more (such as 2, 3, 4, etc.) substituents selected from the group consisting of halogen (including but not limited to F, Cl, Br), -CH₂Cl, -CHCl₂, -CCl₃, -CH₂F, -CHF₂, -CF₃, oxo, -CN, hydroxyl, amino, C1-C6 alkylamino, carboxyl, -NHAc, or substituted or unsubstituted groups which are selected from C1-C6 alkyl, C1-C6 alkoxy, C6-C10 aryl, C3-C8 cycloalkyl, halogenated C6-C10 aryl, 5-10 membered heteroaryl with 1-3 heteroatoms selected from N, S and O, and 5-10 membered heterocyclyl with 1-3 heteroatoms selected from N, S and O, and the substituent of which is selected from halogen, hydroxyl, carboxyl, cyano, C1-C6 alkoxy, and C1-C6 alkylamino;
among the above formula, any of the heteroatoms is selected from the group consisting of B, P, N, S and O.

2. The compound of claim 1, or isomers, optical isomers, hydrates, solvates thereof, or pharmaceutically acceptable salts thereof, wherein 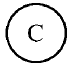 is a divalent group formed by a ring selected from the group consisting of 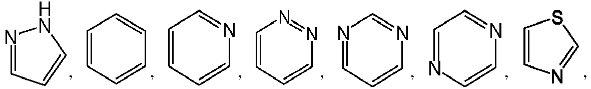 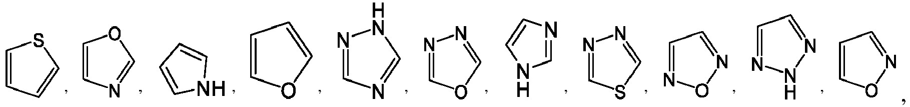 wherein bonding position of the ring can be N or C.

3. The compound of claim 1, wherein 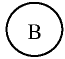 is selected from the group consisting of: 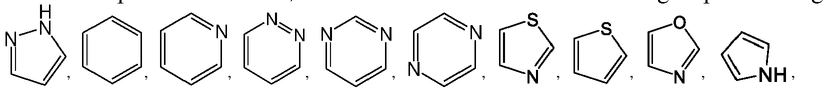 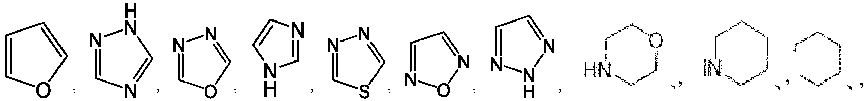 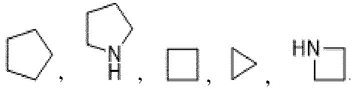 and R₁ and R₂ are each independently selected from the group consisting of H, halogen, substituted or unsubstituted C1-C10 alkyl.

4. The compound of claim 1, wherein 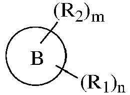 is selected from the group consisting of:

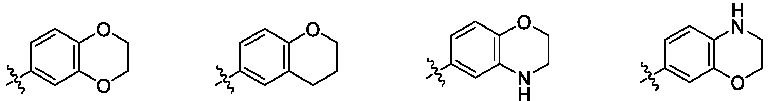

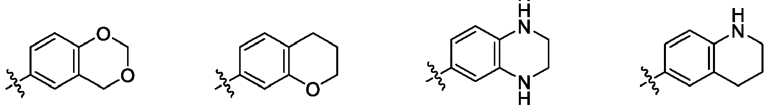

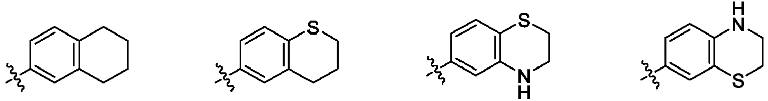

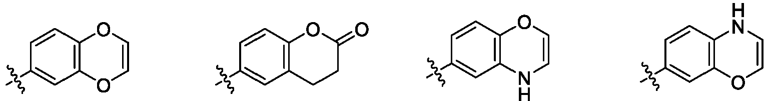

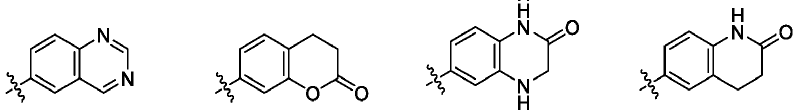

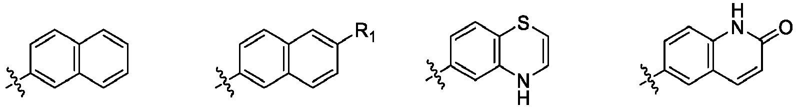

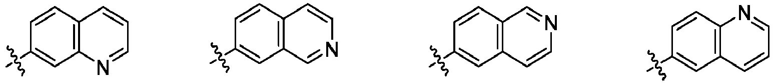

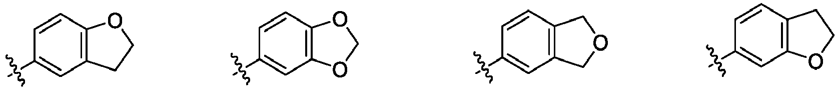

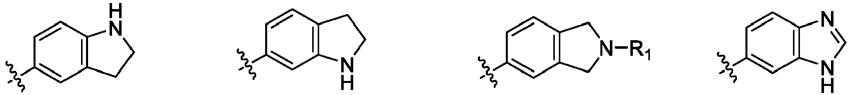

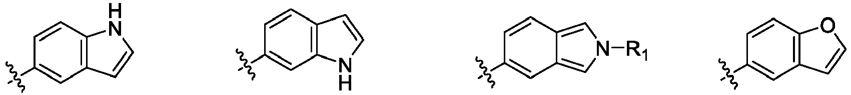

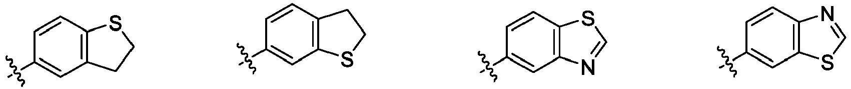

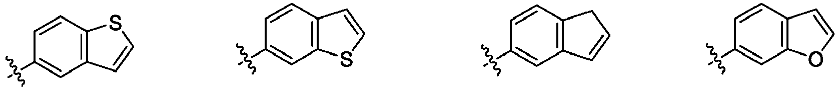

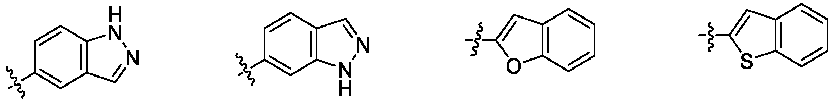

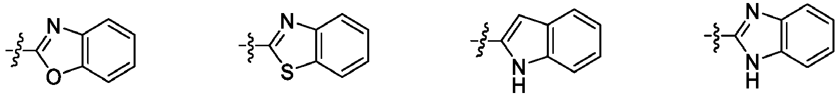

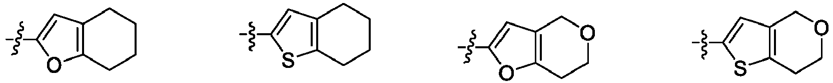

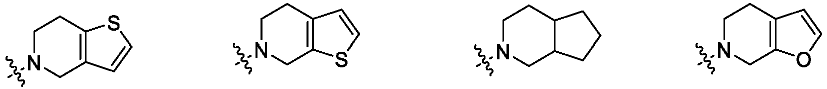

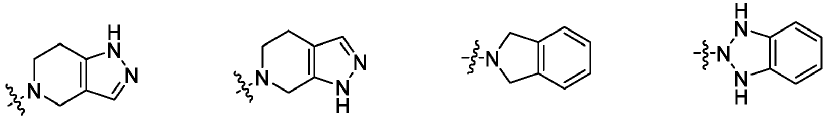

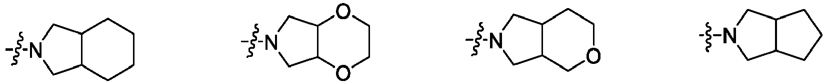

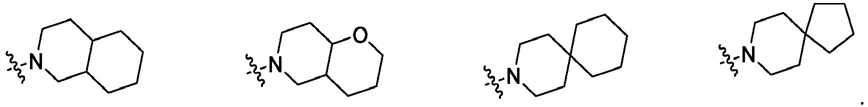

5. The compound of claim 1, wherein 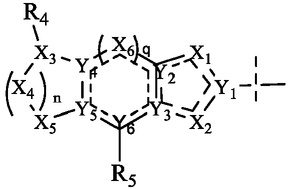 is the structure shown below:

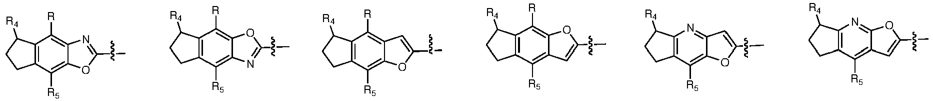

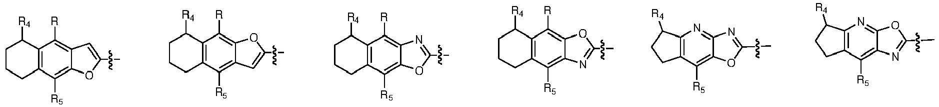

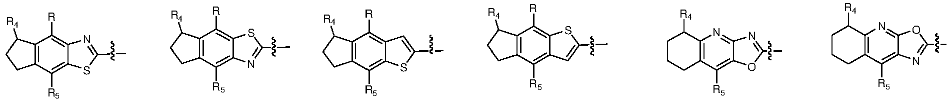

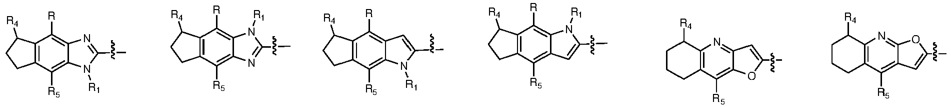

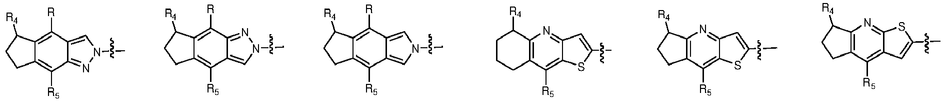

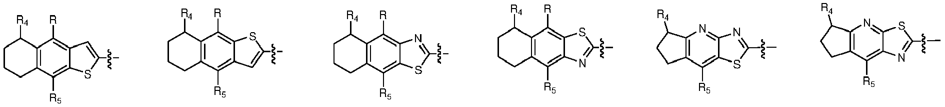

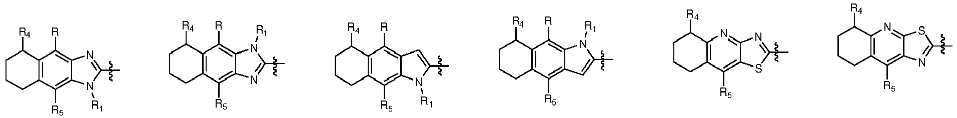

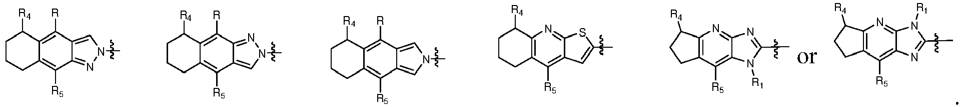

6. The compound of claim 1, or optical isomers, hydrates, solvates thereof, or pharmaceutically acceptable salts thereof, wherein the compound is selected from the group consisting of:

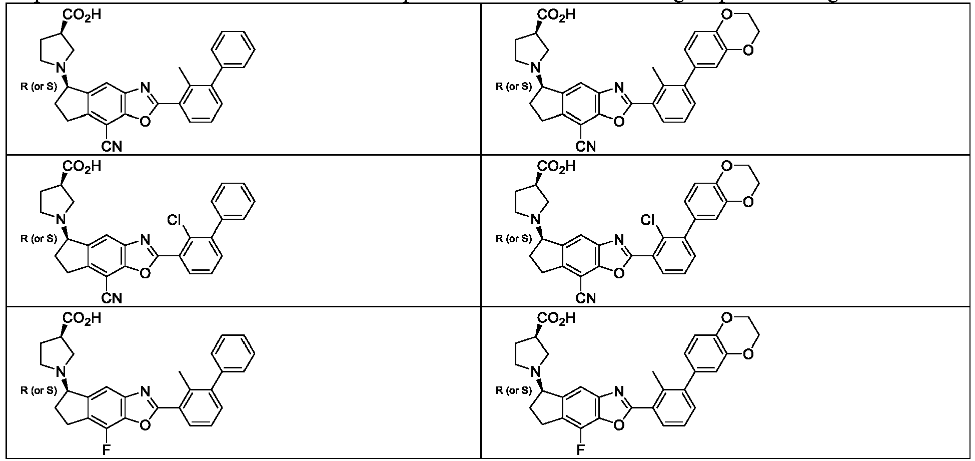

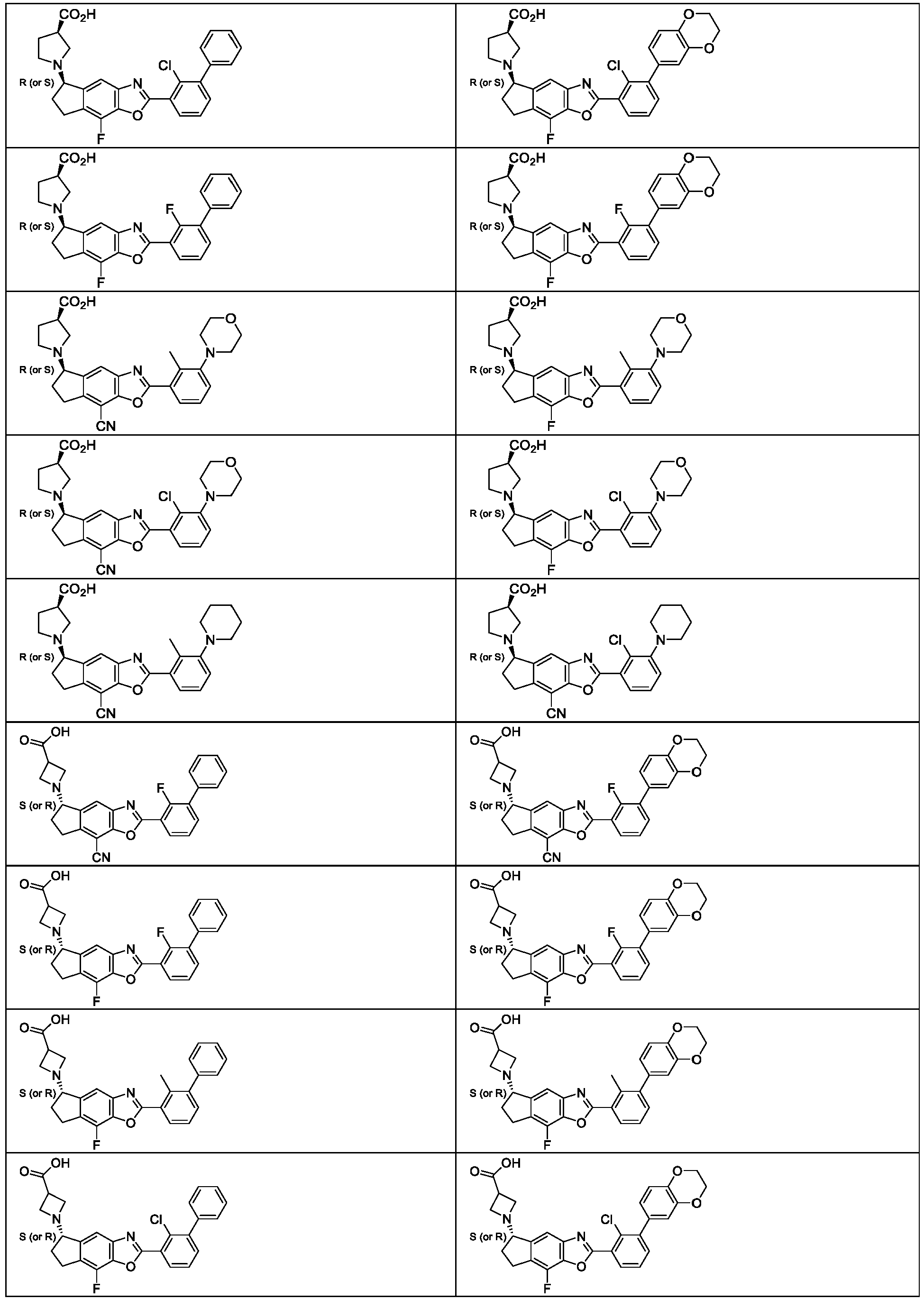

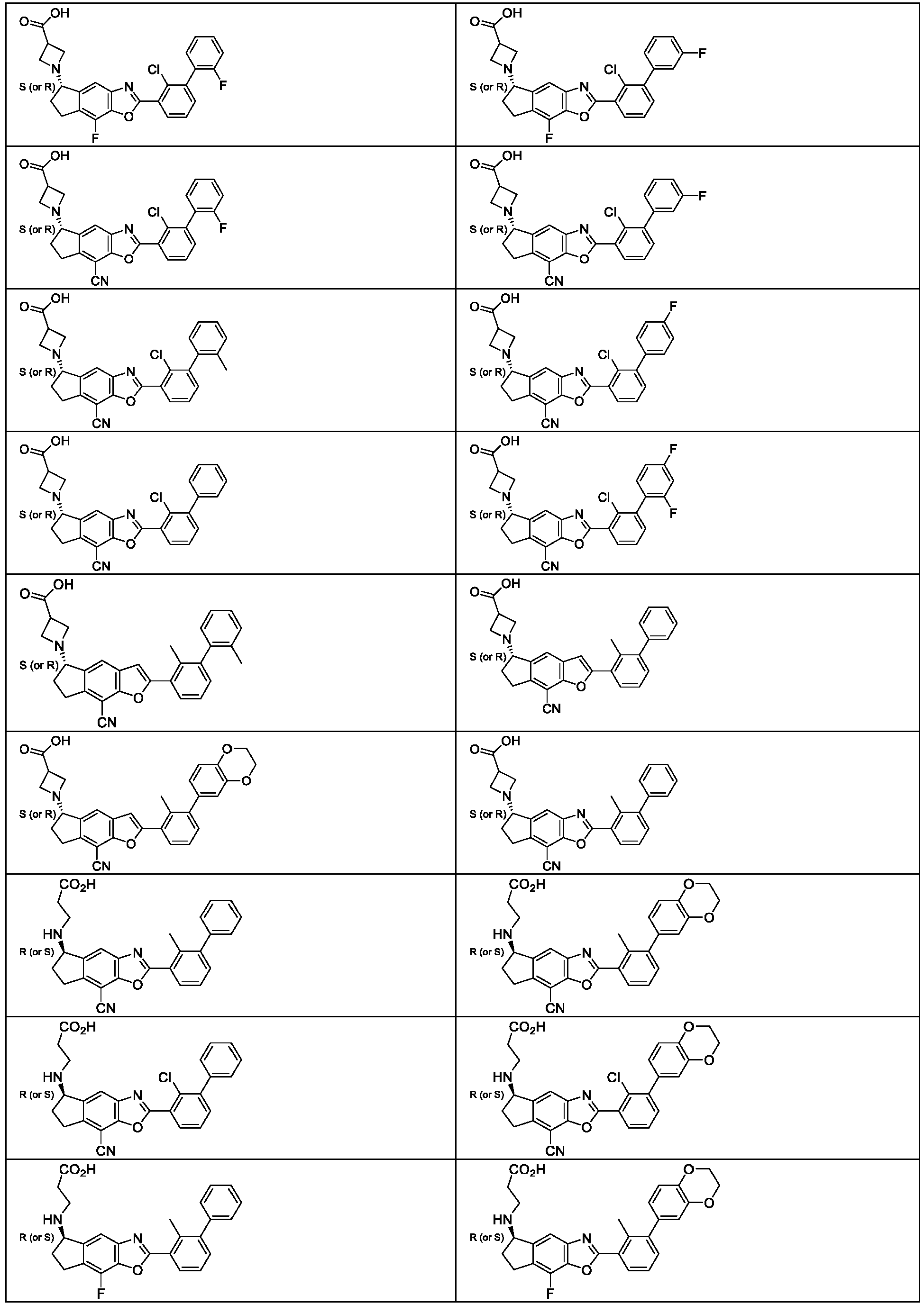

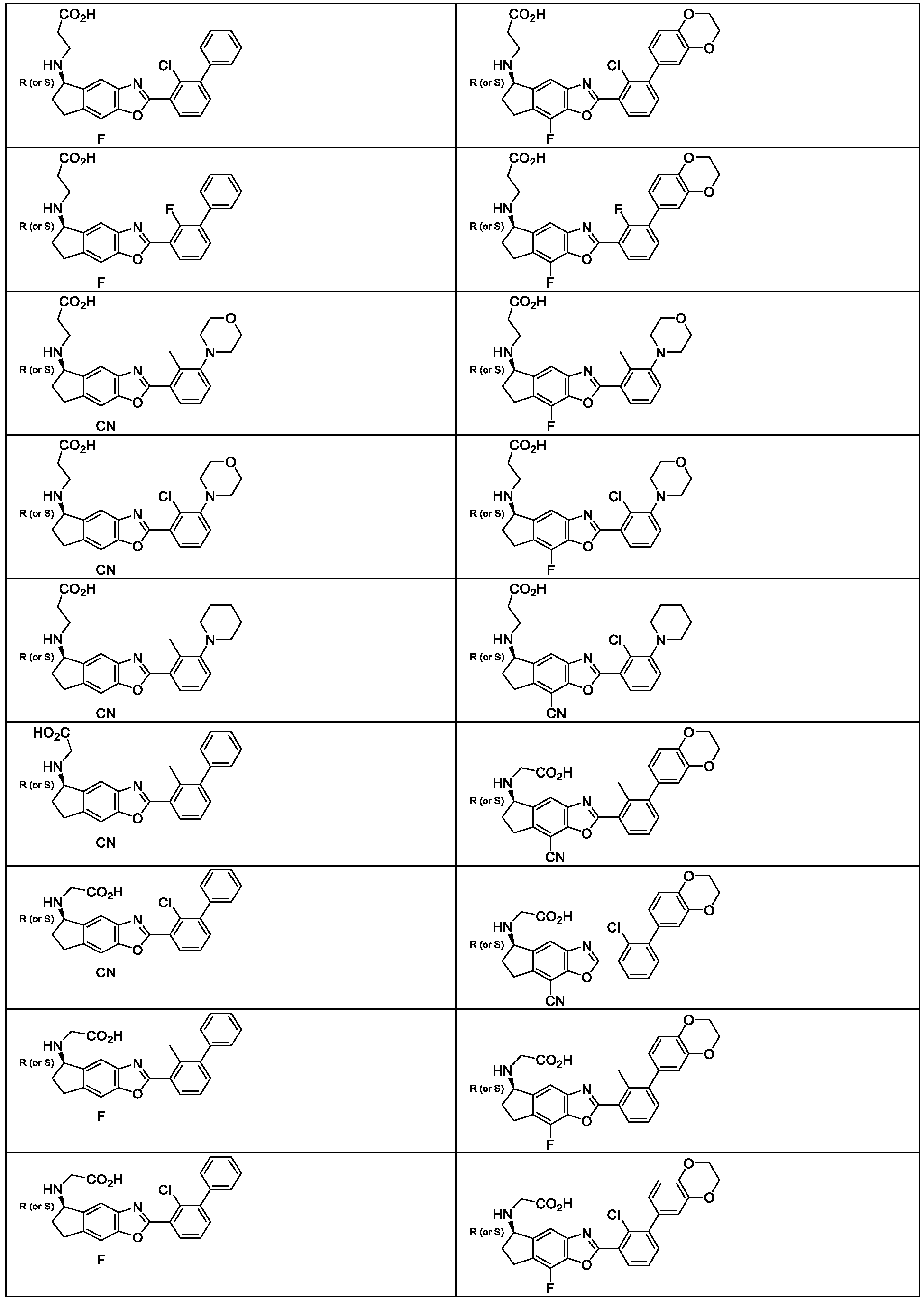

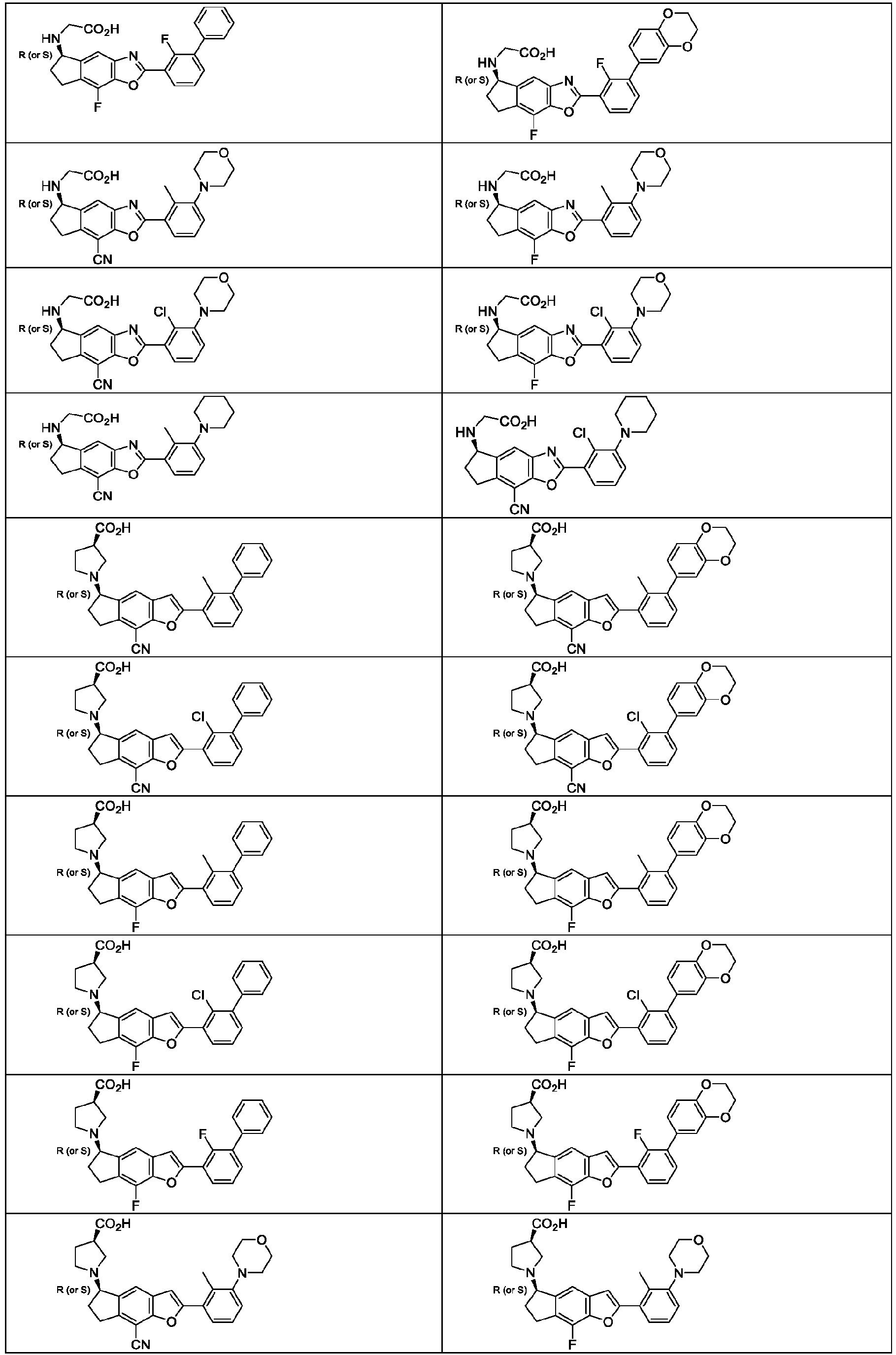

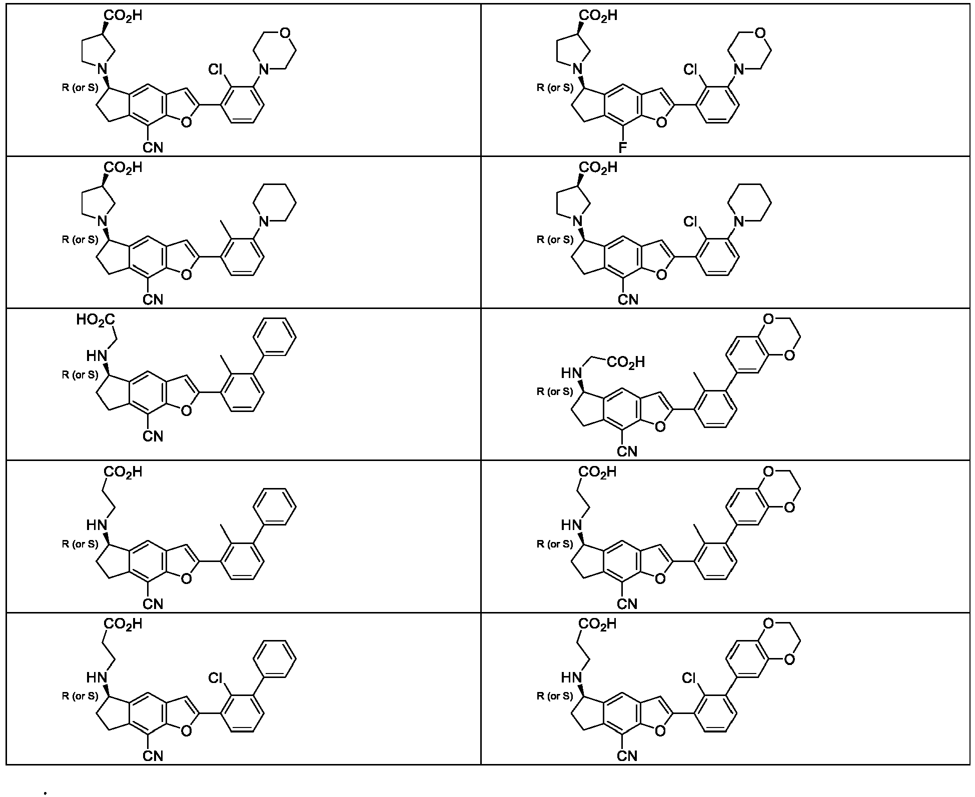

7. A method for preparing compound of formula I of claim 1, wherein the method comprises the steps selected from the synthesis scheme 1 or 2:
synthesis scheme 1: 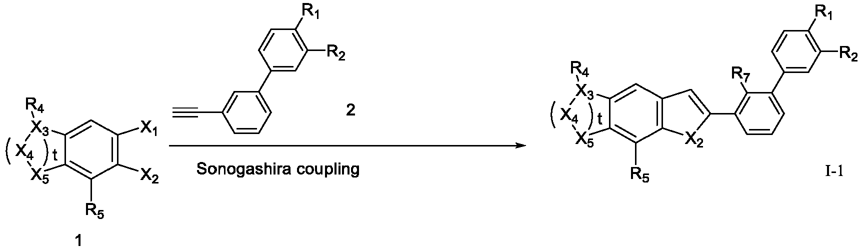
(a) providing the target product 1-1 by Sonogashira coupling reaction catalyzed by palladium catalyst by using intermediate 1 and 2 as raw materials;
preferably, the preparation method of intermediate 1 is as follows: 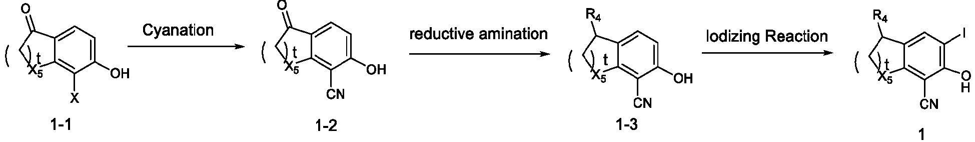
providing intermediate 1-2 by coupling reaction with palladium catalyst and ligand using compound 1-1 as raw material;
(b) providing intermediate **1-3** by first condensation reaction with primary and secondary amines under the catalysis of Lewis acid using **1-2** as the raw material, then conducting reduction reaction by adding appropriate reducing agent; or obtaining the above by directly reductive amination reaction in the presence of reducing agent;
(c) providing intermediate **1** by halogenation reaction catalyzed by Lewis acid using **1-3** as raw material;
synthetic scheme 2: 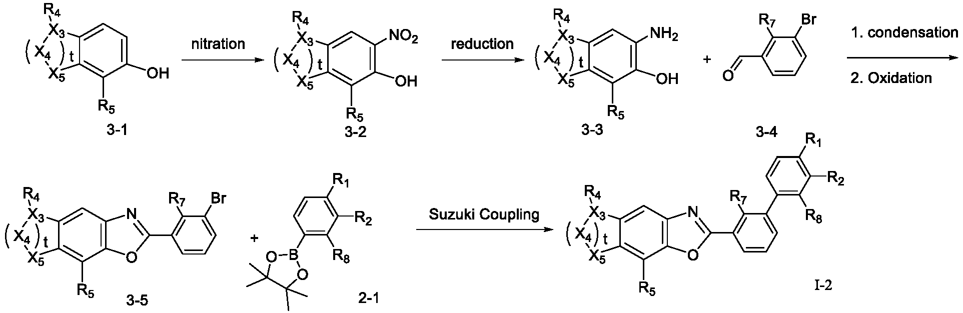
(a) providing intermediate **3-2** by using nitrating agent (such as concentrated sulfuric acid/NaNO₃, concentrated sulfuric acid/fuming nitric acid, etc.) to react with compound **3-1;**
(b) providing intermediate **3-3** by reducing **3-2** under reductive conditions (Pd-C/H₂; zinc powder/ammonium chloride; iron powder/acetic acid, etc.);
(c) providing intermediate **3-5** by condensing **3-3** and **3-4** with condensation agent, then oxidating with appropriate oxidant (such as DDQ);
(d) providing intermediate **1-2** by coupling **3-5** and **3-6** with catalyst and ligand.

8. A pharmaceutical composition, comprises (1) the compound of claim 1 or stereoisomers or tautomers thereof, or pharmaceutically acceptable salts, hydrates or solvates thereof; (2) pharmaceutically acceptable carriers.

9. A use of the compound of claim 1 or stereoisomers or tautomers thereof, or pharmaceutically acceptable salts, hydrates or solvates thereof, or a pharmaceutical composition of claim 7, for the preparation of a pharmaceutical composition for preventing and/or treating diseases related to the activity or expression of PD-1/PD-L1.

10. A PD-1/PD-L1 regulator, comprises the compound of claim 1, or stereoisomers or tautomers thereof, or pharmaceutically acceptable salts, hydrates or solvates thereof.
